# FASCICULE DE BREVET EUROPEEN

(11) **EP 1 269 980 B1**
(45) Date de publication et mention de la délivrance du brevet: **09.02.2005**
(21) Numéro de dépôt: 02291554.0
(22) Date de dépôt: 21.06.2002
(51) Int. Cl.: A61K 7/42

(54) **Solubilisation de derives 1, 3,5 triazine par des esters N-acyles d'acide amine**
Solubilisierung von 1,3,5-Triazinderivaten mittels Estern von N-Acylaminosäuren
Solubilisation of 1,3,5-triazine derivatives using N-acylamino acid esters

(30) Priorité: 26.06.2001 FR 0108426
(43) Date de publication de la demande: 02.01.2003
(73) Titulaire: L'OREAL, 75008 Paris (FR)
(72) Inventeur: Candau, Didier, 91570 Bievres (FR)
(74) Mandataire: Casalonga, Axel

(56) Documents cités:
- EP-A- 0 766 959
- EP-A- 0 813 861
- EP-A- 0 863 145
- EP-A- 0 913 390
- EP-A- 0 928 608
- EP-A- 1 034 778
- EP-A- 1 044 676
- PATENT ABSTRACTS OF JAPAN vol. 1996, no. 02, 29 février 1996 (1996-02-29) & JP 07 277937 A (AJINOMOTO CO INC), 24 octobre 1995 (1995-10-24)
- PATENT ABSTRACTS OF JAPAN vol. 1999, no. 12, 29 octobre 1999 (1999-10-29) & JP 11 189522 A (AJINOMOTO CO INC), 13 juillet 1999 (1999-07-13)

## Description

La présente invention concerne une composition comprenant au moins un dérivé de 1,3,5 triazine ainsi que son utilisation dans ou pour la fabrication de compositions cosmétiques ou dermatologiques destinées à la protection de la peau et/ou des lèvres et/ou des phanères contre le rayonnement ultra-violet, en particulier le rayonnement solaire.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions anti-solaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction du facteur de protection solaire recherché, le facteur de protection solaire (FPS) s'exprimant mathématiquement par le rapport de la dose de rayonnement UV nécessaire pour atteindre le seuil érythématogène avec le filtre UV sur la dose de rayonnement UV nécessaire pour atteindre le seuil érythématogène sans filtre UV.

Les dérivés de 1,3,5-triazine sont particulièrement recherchés dans la cosmétique solaire du fait qu'ils sont fortement actifs dans l'UVB, et même dans l'UV-A pour certains de ces composés selon la nature des substituants mis en jeu. De plus, ils sont photostables, c'est à dire qu'ils se dégradent peu ou pas chimiquement sous l'action du rayonnement UV. Ils sont notamment décrits dans les demandes de brevet US 4367390, EP863145, EP517104, EP570838, EP796851, EP775698, EP878469 et EP933376, et on connaît en particulier :
- la 2,4,6-tris[p-(2'-éthylhexyl-1'-oxycarbonyl)anilino]-1,3,5-triazine ou « Ethylhexyl Triazone » (nom INCI), vendue sous la dénomination commerciale « Uvinul T 150 » par la société BASF,
- la 2-[(p-(tertiobutylamido)anilino]-4,6-bis-[(p-(2'-éthylhexyl-1'-oxycarbonyl)anilino]-1,3,5-triazine ou « Diethylhexyl Butamido Triazone » (nom INCI) vendu sous le nom commercial « UVASORB HEB » par SIGMA 3V,
- la 2,4-bis {[4-2-éthyl-hexyloxy)]-2-hydroxy]-phenyl}-6-(4-méthoxy-phenyl)-1,3,5-triazine ou « Anisotriazine » (nom INCI) vendu sous le nom commercial «TINOSORB S » par CIBA SPECIALTY CHEMICALS.

Dans l'art antérieur, il a été proposé d'utiliser des dérivés de 1,3,5-triazine dans des huiles comme des esters et plus particulièrement des benzoates d'alkyles en C₁₂-C₁₅ ("FINSOL V TN" de chez Finetex), ou des triglycérides et notamment des triglycérides d'acides gras en C₈-C₁₂ ("MIGLYOL 812" de chez Hüls), ou encore des monoalcools ou des polyols gras oxyéthylénés ou oxypropylénés ( « CETIOL HE » de chez Henkel ou « WITCONOL AM» de chez WITCO ).

Cependant il a été observé que le pouvoir photoprotecteur de ces dérivés de triazine en l'absence d'autres filtres solaires est très limité et que leurs propriétés cosmétiques sont généralement jugées insuffisantes.

Le problème posé à la base de la présente invention était d'améliorer l'efficacité photoprotectrice des compositions contenant de tels dérivés de 1,3,5-triazine.

De manière inattendue et surprenante, les inventeurs de la présente demande ont montré que l'utilisation d'au moins un ester choisi parmi les esters N-acylés d'acide aminés de formule :

R'₁(CO)N(R'₂)CH(R'₃)(CH₂)ₙ(CO)OR'₄

dans laquelle :
n est un entier égal à 0,1 ou 2,
R'₁ représente un radical alkyle ou alcényle en C₅ à C₂₁, linéaire ou ramifié,
R'₂ représente un atome d'hydrogène ou un groupe alkyle en C₁ à C₃,
R'₃ représente un radical choisi dans le groupe formé par un atome d'hydrogène, un groupe méthyle, un groupe éthyle, une chaîne alkyle linéaire ou ramifiée en C₃ ou C₄,
R'₄ représente un radical alkyle en C₁ à C₁₀ linéaire ou ramifié, ou un radical alcényle en C₂ à C₁₀ linéaire ou ramifié ou un reste stérol, permet l'obtention d'une composition contenant des dérivés 1,3,5-triazine qui présente des propriétés cosmétiques et photoprotectrices améliorées. Ces esters N-acylés d'acide aminés ainsi que leur procédé de préparation sont décrits dans les demandes de brevets EP 1 044 676 et EP 0 928 608 de la société AJINOMOTO CO.

Le principal avantage de ces esters N-acylés d'acide aminés est qu'ils permettent l'obtention de compositions anti-solaires contenant des dérivés 1,3,5 triazine et qui présentent un facteur de protection solaire supérieur à ceux des compositions de l'art antérieur contenant dérivés 1,3,5 triazine.

Ces compositions possèdent en outre des qualités cosmétiques améliorées. Elles permettent une bonne hydratation de la peau c'est-à-dire que l'on n'observe pas de dessèchement de la peau, ni à l'inverse un toucher trop gras.

La présente invention a donc pour objet une composition contenant au moins un dérivé de 1,3,5-triazine et au moins un ester choisi parmi les esters N-acylés d'acide aminés.

Un autre objet de l'invention consiste en l'utilisation d'une telle composition pour la fabrication de compositions cosmétiques ou dermatologiques.

L'invention a également pour objet un procédé de traitement cosmétique comprenant l'application d'une composition selon la présente invention ainsi que l'utilisation d'au moins un ester N-acylés d'acide aminé de formule

R'₁(CO)N(R'₂)CH(R'₃)(CH₂)ₙ(CO)OR'₄

dans laquelle :
n est un entier égal à 0,1 ou 2,
R'₁ représente un radical alkyle ou alcényle en C₅ à C₂₁, linéaire ou ramifié,
R'₂ représente un atome d'hydrogène ou un groupe alkyle en C₁ à C₃,
R'₃ représente un radical choisi dans le groupe formé par un atome d'hydrogène, un groupe méthyle, un groupe éthyle, une chaîne alkyle linéaire ou ramifiée en C₃ ou C₄,
R'₄ représente un radical alkyle en C₁ à C₁₀ linéaire ou ramifié, ou un radical alcényle en C₂ à C₁₀ linéaire ou ramifié ou un reste stérol dans des compositions antisolaires contenant un dérivé de 1,3,5-triazine en vue d'améliorer le facteur de protection solaire de cette composition.

D'autres objets de l'invention apparaîtront à la lecture de la description et des exemples qui suivent.

La composition objet de l'invention comprend de préférence dans un milieu physiologiquement acceptable,
(i) au moins un dérivé de 1,3,5-triazine, et
(ii) au moins un ester choisi parmi les esters N-acylés d'acide aminés de formule

   R'₁(CO)N(R'₂)CH(R'₃)(CH₂)ₙ(CO)OR'₄
dans laquelle :
n est un entier égal à 0,1 ou 2,
R'₁ représente un radical alkyle ou alcényle en C₅ à C₂₁, linéaire ou ramifié,
R'₂ représente un atome d'hydrogène ou un groupe alkyle en C₁ à C₃,
R'₃ représente un radical choisi dans le groupe formé par un atome d'hydrogène, un groupe méthyle, un groupe éthyle, une chaîne alkyle linéaire ou ramifiée en C₃ ou C₄,
R'₄ représente un radical alkyle en C₁ à C₁₀ linéaire ou ramifié, ou un radical alcényle en C₂ à C₁₀ linéaire ou ramifié ou un reste stérol.

Dans la formule des esters d'aminoacide présentées ci-dessus, le groupement R'₁(CO)- est un groupement acyle d'un acide choisi de préférence dans le groupe formé par l'acide caprique, l'acide laurique, l'acide myristique, l'acide palmitique, l'acide stéarique, l'acide béhénique, l'acide linoléique, l'acide linolénique, l'acide oléique, l'acide isostéarique, l'acide 2-éthylhexanoïque, les acides gras d'huile de coco, les acides gras d'huile de palmiste. Ces acides gras peuvent en outre présenter un groupe hydroxyle. De manière encore plus préférée, il s'agira de l'acide laurique.

La partie -N(R'₂)CH(R'₃)(CH₂)ₙ(CO)- de l'ester d'aminoacide est de préférence choisie parmi les aminoacides suivants : glycine, alanine, valine, leucine, isolcucine, sérine, thréonine, proline, hydroxyproline, β-alanine, acide aminobutyrique, acide aminoca-proique, sarcosine, ou N-méthyl-β-alanine.

De manière encore plus préférée, il s'agira de la sarcosine.

La partie des esters aminoacides correspondant au groupe OR'₄ peut être obtenue à partir des alcools choisis dans le groupe formé par le méthanol, éthanol, propanol, isopropanol, butanol, tert-butanol, isobutanol, 3-méthyl-1-butanol, 2-méthyl-1-butanol, huile de fusel, pentanol, héxanol, cyclohéxanol, octanol, 2-éthylhéxanol, décanol, alcool laurylique, alcool myristique, alcool cétylique, alcool cétostéarylique, alcool stéarylique, akool oléique, alcool béhénylique, alcool de jojoba, alcool 2-héxadécylique, alcool 2-octyldodécanol et alcool isostéarylique.

Ces esters d'acide aminé peuvent en particulier être obtenus à partir de sources naturelles en acides aminés. Dans ce cas, les acides aminés proviennent d'hydrolyse de protéines naturelles végétales (avoine, blé, soja, palme, coco) et conduisent alors nécessairement à des mélanges d'acides aminés qui devront ensuite être estérifiés puis N-acylés. La préparation de tels acides aminés est plus particulièrement décrite dans la demande de brevet FR 2 796 550.

L'ester d'acide aminé plus particulièrement préféré pour son utilisation dans la présente invention est le N-lauroylsarcosinate d'isopropyl de formule :

CH₃-(CH₂)₁₀CO-N(CH₃)-CH₂-COO-CH-(CH₃)₂.

Le dérivé de 1,3,5-triazine répond à la formule (I) suivante : 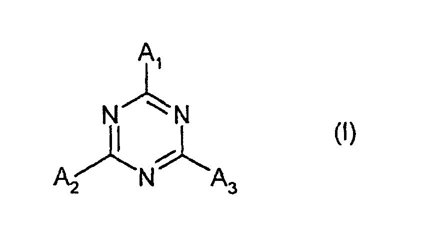 dans laquelle les radicaux A₁, A₂ et A₃, identiques ou différents sont choisis parmi les groupes de formules (II) à (IX) suivantes : 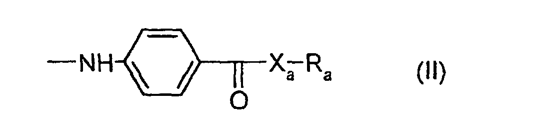 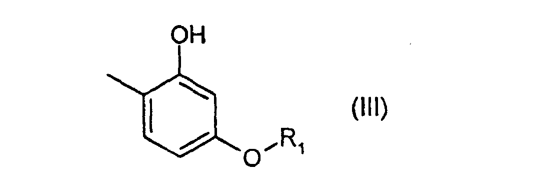 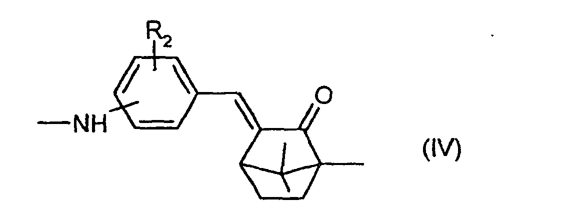 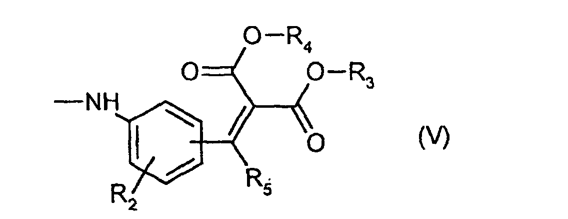 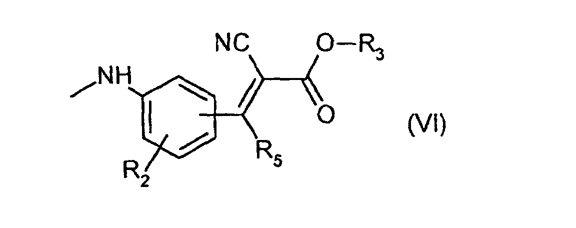 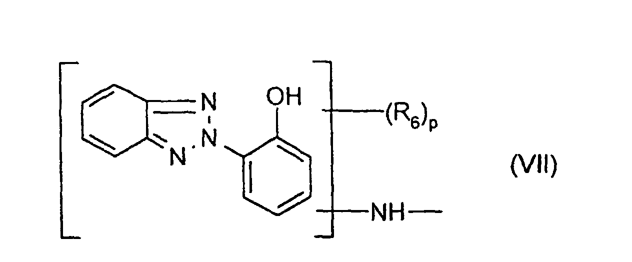 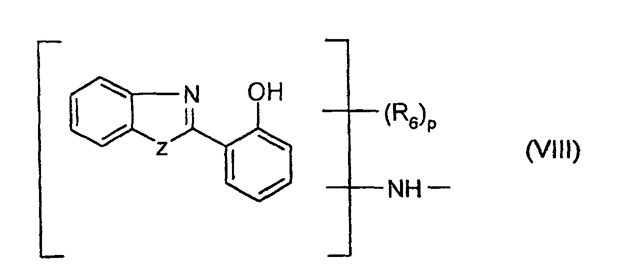 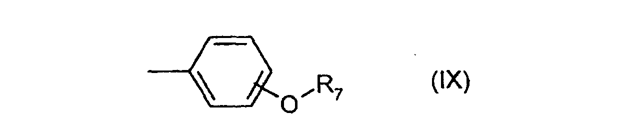 dans lesquelles :
- Xₐ (chacun des Xₐ peut être identique ou différent) représente l'oxygène ou -NH-;
- Rₐ (chacun des Rₐ peut être identique ou différent) est choisi parmi l'hydrogène; un métal alcalin; un radical ammonium éventuellement substitué par un ou plusieurs radicaux alkyles linéaire ou ramifié en C₁-C₁₈ ou hydroxyalkyles linéaire ou ramifié en C₁-C₁₈; un radical alkyle linéaire ou ramifié en C₁-C₁₈, de préférence en C₆-C₁₂; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé ; un radical de formule (X), (XI) ou (XII) suivantes : 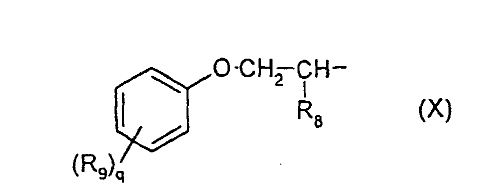 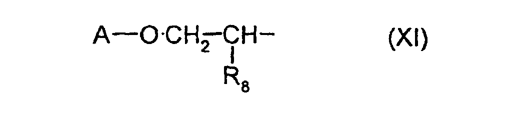 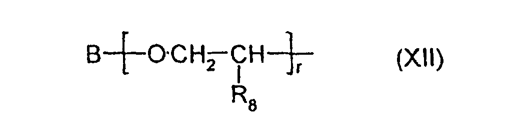
dans lesquelles :
- R₈ est l'hydrogène ou un radical méthyle;
- R₉ est un radical alkyle en C₁-C₉;
- q est un nombre entier égal à 0; 1; 2; 3;
- r est un nombre entier égal à 1; 2; 3; 4; 5; 6; 7; 8; 9; 10;
- A est un radical alkyle en C₄-C₈ ou un radical cycloalkyle en C₅-C₈;
- B est choisi parmi : un radical alkyle linéaire ou ramifié en C₁-C₈; un radical cycloalkyle en C₅-C₈; un radical aryle éventuellement substitué par un ou plusieurs radicaux alkyles en C₁-C₄.
- R₁ désigne un radical alkyle en C₃-C₁₈ ; un radical alcényle en C₂-C₁₈ ; un reste de formule -CH₂-CH(OH)-CH₂-OT₁ où T₁ est un atome d'hydrogène ou un radical alkyle en C₁-C₈ ; un reste de formule (XIII) suivante : 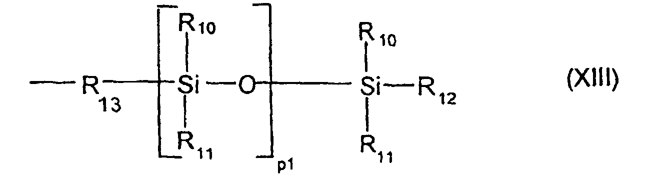 dans laquelle :
   - R₁₃ désigne une liaison covalente ; un radical alkyle linéaire ou ramifié en C₁-C₄ ou bien un radical de formule -Cₘ₁H₂ₘ₁- ou-Cₘ₁H₂ₘ₁-O- où m₁ est un entier égal à 1; 2; 3; 4 ;
   - p₁ est un entier égal à 0; 1; 2; 3; 4; 5 ;
   - les radicaux R₁₀, R₁₁ et R₁₂, identiques ou différents, désignent un radical alkyle en C₁-C₁₈ ; un radical alcoxy en C₁-C₁₈ ou un radical de formule : 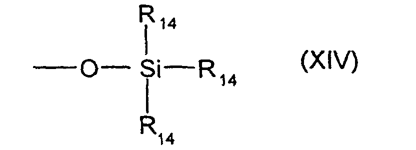
   où R₁₄ est un radical alkyle en C₁-C₅.
   - R₂ désigne un atome d'hydrogène, un radical alkyle en C₁-C₄ linéaire ou ramifié ou un radical alkoxy en C₁-C₄ ;
   - R₃ et R₄, identiques ou différents désignent un radical alkyle linéaire ou ramifié en C₁-C₂₀;
   - R₅ représente un atome d'hydrogène ou un radical phényle éventuellement substitué par un halogène ou par un radical alkyle en C₁-C₄ ou par un radical alcoxy en C₁-C₄ ;
   - R₆ est un radical alkyle en C₁-C₈ linéaire ou ramifié, alkoxy en C₁-C₃ étant entendu que, dans ce dernier cas, deux R₆ adjacents d'un même noyau aromatique peuvent former ensemble un groupement alkylidène dioxy dans lequel le groupe alkylidène contient de 1 à 2 atomes de carbone, OH, NHCOCH₃ ou NH₂,
   - R₇ désigne un atome d'hydrogène, un radical alkyle en C₁-C₁₀, un radical de formule : -(CH₂CHR₅-O)ₙ₁R₈ où n1 est un nombre de 1 à 16, ou bien un radical de structure -CH₂-CH-(OH)-CH₂OT₁ avec R₈ et T₁ ayant la même signification indiquée ci-dessus.
   - Z représente l'oxygène, le soufre, -NH- ou -NR₃ - avec R₃ représentant un radical alkyle en C₁-C₂₀ linéaire ou ramifié;
   - p est 0, 1, 2 ou 3,

A₁ peut également être un halogène, un radical -N(R₃)₂, les deux R₃ pouvant former ensemble un cycle de 4 ou 5 atomes de carbone, ou un groupe -OR₃, R₃ ayant la même définition qu'au dessus.

Une première famille plus particulièrement préférée de dérivés de 1,3,5-triazine, notamment décrite dans le document EP-A-0 517 104, est celle des 1,3,5-triazines répondant à la formule (I) dans laquelle les A₁, A₂ et A₃ sont de formule (II) et présentant l'ensemble des caractéristiques suivantes :
- l'un des groupements Xₐ-Rₐ représente le radical -NH-Rₐ avec Rₐ choisi parmi : un radical cycloalkyle en C₅-C₁₂ éventuellement substitué par un ou plusieurs radicaux alkyles en C₁-C₄; un radical de formule (X), (XI) ou (XII) ci-dessus dans lesquelles :
   - B est un radical alkyle en C₁-C₄ ;
   - R₉ est le radical méthyle ;
- les 2 autres Xₐ-Rₐ représentent le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (X), (XI) ou (XII) ci-dessus dans lesquelles :
   - B est un radical alkyle en C₁-C₄;
   - R₉ est le radical méthyle.

Une deuxième famille plus particulièrement préférée de dérivés de 1,3,5-triazine, notamment décrite dans le document EP-A-0 570 838, est celle des 1,3,5-triazines répondant à la formule (I) dans laquelle les A₁, A₂ et A₃ sont de formule (II) et présentant l'ensemble des caractéristiques suivantes :
- un ou deux Xₐ-Rₐ représente le radical -NH-Rₐ , avec Rₐ choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄; un radical de formule (X), (XI) ou (XII) ci-dessus dans lesquelles :
   - B est un radical alkyle en C₁-C₄ ;
   - R₉ est le radical méthyle ;
le ou les deux autres Xₐ-Rₐ étant le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (X), (XI) ou (XII) ci-dessus dans lesquelles :
- B est un radical alkyle en C₁-C₄;
- R₉ est le radical méthyle.

Une 1,3,5-triazine particulièrement préférée de cette deuxième famille est la 2-[(p-(tertiobutylamido)anilino]-4,6-bis-[(p-(2'-éthylhexyl-1'-oxycarbonyl)anîlino]-1,3,5-triazine ou « Diethylhexyl Butamido Triazone » vendue sous le nom commercial « UVASORB HEB » par SIGMA 3V et répondant à la formule suivante : 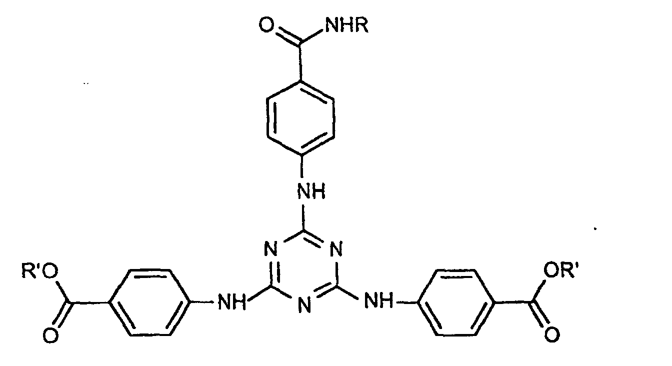 dans laquelle R' désigne un radical éthyl-2 hexyle et R désigne un radical tert-butyle.

Une troisième famille préférée de composés utilisables dans le cadre de la présente invention, et qui est notamment décrite dans le document US 4,724,137, est celle des 1,3,5-triazines répondant à la formule (I) dans laquelle les A₁, A₂ et A₃ sont de formule (II) et présentant l'ensemble des caractéristiques suivantes :
- Xₐ sont identiques et représentent l'oxygène;
- Rₐ, identiques ou différents et représentent un radical alkyle en C₆-C₁₂ ou un radical polyoxyéthyléné comprenant de 1 à 6 unités d'oxyde d'éthylène et dont le groupe OH terminal est méthylé.

Une 1,3,5-triazine particulièrement préférée de cette troisième famille est la 2,4,6-tris[p-(2'-éthylhexyl-1'-oxycarbonyl)anilino]-1,3,5-triazine ou « Ethylhexyl Triazone » vendue notamment sous la dénomination commerciale de "UVINUL T 150" par la Société BASF et répond à la formule suivante : 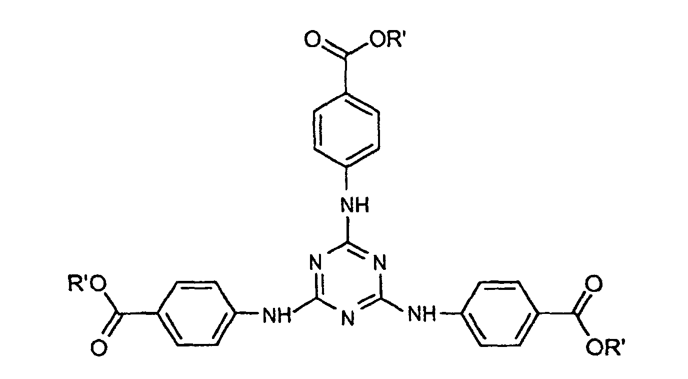 dans laquelle R' désigne un radical 2-éthyl hexyle.

Une quatrième famille préférée de composés utilisables dans le cadre de la présente invention, et qui est notamment décrite dans la demande de brevet EP-A-0775698 est celle des 1,3,5-triazines répondant à la formule (I) dans laquelle les A₁ et A₂ sont de formule (III) et A₃ est de formule (IX) et présentant l'ensemble des caractéristiques suivantes : R₁, identiques ou différents, désignent un radical alkyle en C₃-C₁₈ ; un radical alcényle en C₂-C₁₈ ou bien un reste de formule -CH₂-CH(OH)-CH₂-OT₁ où T₁ est un atome d'hydrogène ou un radical alkyle en C₁-C₈ ; R₇ désigne un atome d'hydrogène, un radical alkyle en C₁-C₁₀.

Une 1,3,5-triazine particulièrement préférée de cette quatrième famille est la 2,4-bis {[4-2-éthyl-hexyloxy)]-2-hydroxy]-phenyl}-6-(4-méthoxy-phenyl)-1,3,5-triazine ou « Anisotriazine » vendue sous le nom commercial «TINOSORB S » par CIBA SPECIALTY CHEMICALS et répond à la formule suivante : 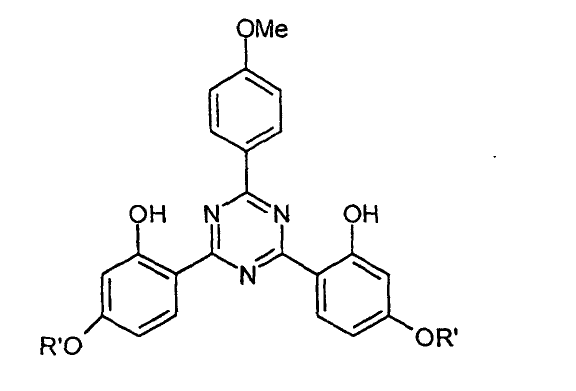 dans laquelle R' désigne un radical éthyl-2 hexyle.

Une cinquième famille préférée de composés utilisables dans le cadre de la présente invention, et qui est notamment décrite dans les demandes de brevet EP507691, EP507692, EP790243 et EP944624 et dont le contenu technique est intégré totalement à la présente description est celle des 1,3,5-triazines répondant à la formule (I) dans laquelle les A₁, A₂ et A₃ sont de formules (VII) à (XI) citées précédemment.

A titre d'exemples de ces composés de formule utilisables, on peut citer :
- la 2,4,6-tris-(4'-amino benzalmalonate de diisobutyle)-s-triazine,
- la 2,4,6-tris-(4'-amino benzalmalonate de di(2-éthylhexyle))-s-triazine,
- la 2,4,6-tris-(4'-amino benzalmalonate de di(2-éthylhexyle))-6-chloro-s-triazine,
- la 2,4,6-tris-(4'-amino benzalmalonate de di(2-éthylhexyle))-6-(4'-aminobenzoate de 2-éthylhexyle)-s-triazine,
- la 2,4,6-tris-(4'-amino benzalmalonate de diisobutyle)-6-butoxy-s-triazine,
- la 2,4,6-tris-(4'-amino benzalmalonate de diisobutyle)-6-(2-éthylhexylamino)-s-triazine,
- la 2,4-bis-(4'-aminobenzylidène camphre)-6-(2-éthylhexylamino)-s-triazine,
- la 2,4-bis-(4'-aminobenzylidène camphre)-6-(4'-amino benzalmalonate de diisobutyle)-s-triazine,
- la 2,4,6-tris(4'-amino benzalmalonate de diéthyle)-s-triazine,
- la 2,4,6-tris(4'-amino benzalmalonate de diisopropyle)-s-triazine,
- la 2,4,6-tris(4'-amino benzalmalonate de diméthyle)-s-triazine,
- la 2,4,6-tris(α-cyano-4-aminocinnamate d'éthyle)-s-triazine.
- la 2,4,6-tris[(3'-benzotriazol-2-yl-2'-hydroxy-5'-methyl) phenylamino]-s-triazine,
- 2,4,6-tris[(3'-benzotriazol-2-yl-2'-hydroxy-5'-ter-octyl) phénylamino]-s-triazine.

Une 1,3,5-triazine particulièrement préférée de cette cinquième famille est la 2,4,6-tris-(4'-amino benzalmalonate de diisobutyle)-s-triazine qui répond à la formule suivante :

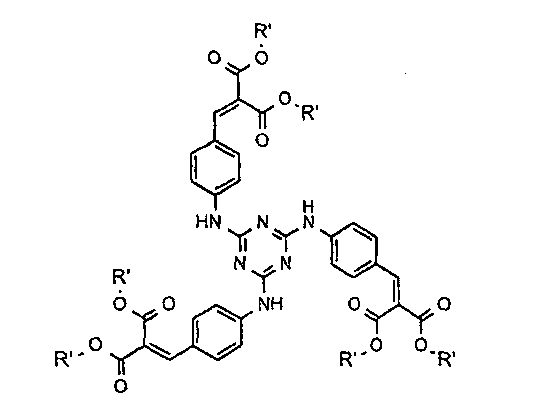

Les compositions selon la présente invention comprennent de préférence, dans un milieu physiologiquement acceptable de 0,05 à 15%, de préférence de 0,1 à 10 % de dérivés 1,3,5 triazine en poids par rapport au poids total de ladite composition.

Les compositions selon la présente invention comprennent de préférence, dans un milieu physiologiquement acceptable, de 0,1 à 50%, de préférence de 1 à 30 % d'esters N-acylés d'acides aminés en poids par rapport au poids total de la composition.

Ladite composition selon la présente invention est de préférence une composition cosmétique contenant outre le dérivé 1,3,5 triazine en tant que filtre organique, au moins un autre filtre organique complémentaire actif dans l'UV-A et/ou l'UV-B (absorbeur) hydrosolubles, liposolubles ou insolubles dans les solvants cosmétiques couramment utilisés.

Ces filtres UV organiques sont choisis parmi les dérivés du dibenzoylméthane, les dérivés cinnamiques, les anthranilates ; les dérivés salicyliques, les dérivés du camphre ; les dérivés de la benzophénone ; les dérivés de β,β-diphénylacrylate, les dérivés de benzotriazole ; les dérivés de benzalmalonate ; les dérivés de benzimidazole ; les imadazolines ; les dérivés bis-benzoazolyle tels que décrits dans les brevets EP669323 et US 2,463,264 ; les dérivés de l'acide p-aminobenzoïque (PABA) ; les dérivés de méthylène bis-hydroxyphényl benzotriazole tels que décrits dans les demandes US5,237,071, US5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649, les 4,4-diarylbutadiènes tels que ceux décrits dans les demandes de brevet EP0967200 et DE19755649 (faisant partie intégrante du contenu de la description).

Les dérivés de l'acide para-benzoïque utilisables dans les compositions selon la présente invention sont les suivants, leur nom INCI est ajouté entre parenthèses :
- l'acide p-aminobenzoïque (PABA),
- l'acide 2-éthyl p-aminobenzoïque (Ethyl PABA)
- (Ethyl Dihydroxypropyl PABA) vendu sous le nom Amerscreen-P par la société Amerchol
- le p-diméthylaminobenzoate de 2-éthylhexyle (Ethylhexyl diméthyl PABA) vendu notamment sous le nom commercial "ESCALOL 507" par la société ISP
- le p-aminobenzoate de glycérol (GlycerylPABA),
- le p-aminobenzoate éthoxylé (25 moles) (PEG-25PABA) vendu sous le nom commercial "UNIVUL P25" par la société BASF,
- le p-aminobenzoate d'éthyle N-proproxylé.

Les dérivés salicyliques utilisables dans les compositions selon la présente invention sont les suivants, leur nom INCI est ajouté entre parenthèses :
- le salicylate d'homomenthyle (homosalate) vendu sous le nom commercial "EUSOLEX HMS" par la société RONA/EM INDUSTRIES,
- le salicylate de 2-éthylhexyle (Ethylhexyl Salicylate) vendu sous le nom commercial "NEO HELIOPAN OS" par la société HAARMANN et REIMER,
- le (Dipropyleneglycol Salicylate) vendu sous le nom commercial "DIPSAL" par la société SCHER,
- le salicylate de triéthanolamine (TEA Salicylate) vendu sous le nom commercial "NEO HELIOPAN TS" par la société HAARMANN et REIMER,
- le salicylate de 4-isopropylbenzyle.

Les dérivés du dibenzoylméthane utilisables dans les compositions selon la présente invention sont les suivants :
- le 2-méthyldibenzoylméthane,
- le 4-méthyldibenzoylméthane,
- le 4-isopropyldibenzoylméthane,
- le 4-tert-butyldibenzoylméthane,
- le 2,4-diméthyldibenzoylméthane,
- le 2,5-diméthyldibenzoylméthane,
- le 4,4'-diisopropyldibenzoylméthane,
- le 4,4'-diméthoxydibenzoylméthane,
- le 4-tert-butyl-4'-méthoxydibenzoylméthane,
- le 2-méthyl-5-isopropyl-4'-méthoxydibenzoylméthane,
- le 2-méthyl-5-tert-butyl-4'-méthoxydibenzoylméthane,
- le 2,4-diméthyl-4'-méthoxydibenzoylméthane,
- le 2,6-diméthyl-4-tert-butyl-4'-méthoxydibenzoylméthane.

Parmi les dérivés du dibenzoylméthane mentionnés ci-dessus, on préfère en particulier, le 4-tert-butyl-4'-méthoxydibenzoylméthane (Butyl methoxydibenzoylméthane), vendu notamment sous la dénomination commerciale de "PARSOL® 1789" par la société HOFFMANN-LAROCHE.

Un autre dérivé du dibenzoylméthane préféré selon la présente invention est le 4-isopropyldibenzoylméthane (Isopropyl Dibenzoylméthane) vendu sous la dénomination de "EUSOLEX® 8020" par la société MERCK
- Les dérivés cinnamiques utilisables dans les compositions selon la présente invention sont les suivants :
- le 4-méthoxycinnamate de 2-éthylhexyle (Ethylhexyl Methoxycinnamate) vendu sous le nom commercial "PARSOL MCX" par la société HOFFMANN LA ROCHE,
- l'isopropyl Methoxy cinnamate,
- le 4-méthoxycinnamate d'isoamyle (Isoamyl methoxy cinnamate) vendu sous le nom commercial "NEO HELIOPAN E 1000" par la société HAARMANN et REIMER,
- le cinoxate,
- le 4-méthoxycinnamate de diéthanolamine, (DEA methoxycinnamate),
- le diisopropylcinnamate de méthyle (Diisopropyl Methylcinnamate),
- (Glyceryl Ethylhexanoate dimethoxycinnamate).

Les dérivés β,β'-diphénylacrylate utilisables dans les compositions selon la présente invention sont les suivants :
- le 2-cyano-3,3-diphénylacrylate de 2-éthylhexyle (Octocrylene) vendu sous le nom commercial "UVINUL N539" par la société BASF,
- le 2-cyano-3,3-diphénylacrylate d'éthyle (Etocrylene) vendu sous le nom commercial "UVINUL N35" par la société BASF.

Les dérivés de la benzophénone utilisables dans les compositions selon la présente invention sont les suivants :
- la 2,4-dihydroxybenzophénone (benzophénone-1), produit vendu sous le nom UVINUL® 400 par BASF ;
- la 2,2',4,4'-tétrahydroxybenzophénone (benzophénone-2), produit vendu sous le nom UVINUL® D50 par BASF ;
- la 2-hydroxy-4-méthoxybenzophénone encore appelée oxybenzone (benzophénone-3), produit vendu sous le nom UVINUL® M40 par BASF ;
- l'acide 2-hydroxy-4-méthoxybenzophénone-5-sulfonique, encore appelé sulisobenzone (benzophénone-4), produit vendu sous le nom UVINUL® MS40 par BASF ; ainsi que sa forme sulfonate de sodium (benzophénone-5);
- la 2,2'-dihydroxy-4,4'-diméthoxybenzophénone (benzophénone-6), produit vendu sous le nom HELISORB® 11 par NORQUAY ;
- la 5-chloro-2-hydroxybenzophénone (benzophénone-13) ;
- la 2,2'-dihydroxy-4-méthoxybenzophénone, encore appelée dioxybenzone ou benzophénone-8, produit vendu sous le nom SPECTRA-SORB® UV-24 par AMERICAN CYANAMID ;
- le sel disodique du diacide 2,2'-dihydroxy-4,4'-diméthoxybenzophénone-5,5'-disulfonique (benzophénone-9), produit vendu sous le nom UVINUL® DS49 par BASF ;
- la 2-hydroxy-4-méthoxy-4'-méthylbenzophénone (benzolphénone-7) ;
- la 2-hydroxy-4-(octyloxy)benzophénone (benzophénone-12).

Les dérivés du benzylidène camphre utilisables dans les compositions selon la présente invention sont les suivants :
- le 3-benzylidène-d,1-camphre (3-Benzylidene Camphor) fabriqué sous le nom commercial "MEXORYL SD" par CHIMEX,
- le 3-(4'-méthylbenzylidène)-d,l-camphre (4-methylbenzylidene Camphor) vendu sous le nom commercial "EUSOLEX 6300" par MERCK,
- (Benzylidene Camphor Sulfonic acid) fabriqué sous le nom commercial "MEXORYL SL" par CHIMEX,
- (Camphor Benzalkonium Methosulfate) fabriqué sous le nom commercial "MEXORYL SO" par CHIMEX,
- (Terephtalylidene Dicamphor Sulfonic acid) fabriqué sous le nom commercial "MEXORYL SX" par CHIMEX,
- (Polyacrylamidomethyl Benzylidene Camphor) fabriqué sous le nom commercial "MEXORYL SW" par CHIMEX

Les dérivés du benzimidazole utilisables dans les compositions selon la présente invention sont les suivants :
- l'acide 2-phényl benzimidazolyle-5 sulfonique vendu sous le nom commercial "EUSOLEX 232" par la société MERCK
- le benzimidazilate vendu sous le nom commercial "NEO HELIOPAN AP" par la société HAARMANN et REIMER.

Les dérivés de benzotriazole utilisables dans les compositions selon la présente invention sont les suivants :
- le drometrizole trisiloxane vendu sous le nom « SILATRIZOLE » par RHODIA CHIMIE,
- le méthylène bis-benzotriazolyl tetramethylbutylphénol, vendu sous forme solide sous le nom commercial « MIXXIM BB/100 » par FAIRMOUNT CHEMICAL ou sous forme micronisée en dispersion aqueuse sous le nom commercial « TINOSORB M » par CIBA SPECIALTY CHEMICALS.

Parmi les anthranilates utilisables selon la présente invention, on peut citer tout particulièrement l'anthranilate de menthyle (Menthyl Anthranilate) vendu sous le nom commercial "NEO HELIOPAN MA®" par la société HAARMANN et REIMER.

Parmi les dérivés d'imidazoline utilisables selon la présente invention, on peut citer tout particulièrement l'ethylhexyl dimethoxybenzylidene Dioxoimidazoline propionate.

Parmi les dérivés du benzalmalonate utilisables selon la présente invention, on peut citer les polyorganosiloxanes à fonction benzalmalonate tel que le produit vendu sous le nom commercial "PARSOL SLX" par la société HOFFMANN LAROCHE.

Les filtres UV organiques préférés au sens de la présente invention sont choisis parmi les composés suivants :
- Ethylhexyl Salicylate,
- Octocrylene,
- Phenylbenzimidazole Sulfonic Acid,
- 4-Methylbenzylidene camphor,
- Benzimidazilate,
- Terephthalylidene Dicamphor Sulfonic,.
- Benzophenone-3,
- Benzophenone-4,
- Benzophenone-5,
- Méthylène bis-Benzotriazolyl Tetramethylbutylphenol,
- Drometrizole Trisiloxane
- et leurs mélanges.

Ladite composition peut en outre comprendre des nacres, des pigments, ou bien encore des nanopigments (taille moyenne des particules primaires: généralement entre 5 nm et 100 nm, de préférence entre 10 nm et 50 nm) d'oxydes métalliques enrobés ou non, comme par exemple des nanopigments d'oxyde de titane (amorphe ou cristallisé sous forme rutile et/ou anatase), de fer, de zinc, de zirconium ou de cérium et leurs mélanges qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions de l'invention peuvent comprendre en outre des adjuvants cosmétiques classiques notamment choisis parmi les corps gras, les solvants organiques autres que ceux utilisés spécifiquement dans le cadre de la présente invention, les émulsionnants, les épaississants ioniques ou non ioniques, les adoucissants, les antioxydants, les agents anti-radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les anti-inflammatoires, les antagonistes de substance P, les charges, les polymères, les propulseurs, les agents alcalinisants ou acidifiants, les colorants ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone volatiles ou non, les isoparaffines, les polyoléfines, les huiles fluorées et perfluorées, les dérivés alkyles de l'acide benzoïque et del'acide hydrobenzoïque. De même, les cires peuvent être choisies parmi les cires animales, fossiles, végétales, minérales ou de synthèse connues en soi.

Parmi les solvants organiques on peut citer les alcools et polyols inférieurs.

Bien entendu, l'homme de l'art veillera à choisir ce ou ces éventuels composés complémentaires et/ou leurs quantités de manière telle que les propriétés avantageuses, en particulier l'augmentation du facteur de protection des dérivés de 1,3,5triazine dans l'ester N-acylé d'acide aminé, ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau (H/E) ou eau-dans-huile (E/H).

Ces compositions peuvent se présenter en particulier sous forme d'émulsion, simple ou complexe : double (H/E ou E/H) ou triple (E/H/E ou H/E/H) telle qu'une crème, un lait, un gel ou un gel crème ; de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des ongles, des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

La présente invention concerne encore l'utilisation d'une composition selon la présente invention dans ou pour la fabrication de compositions cosmétiques ou dermatologiques destinées à la protection de la peau et/ou des lèvres et/ou des phanères (cils, sourcils, cheveux et ongles) contre le rayonnement ultraviolet, en particulier le rayonnement solaire, ainsi qu'un procédé de traitement cosmétique, caractérisé en ce qu'il consiste à appliquer sur la peau, les lèvres, ou les phanères, une composition selon la présente invention.

Les exemples qui suivent illustrent l'invention sans en limiter la portée.

### EXEMPLE

On a préparé deux compositions anti-solaire (C1 conforme à l'invention et C2 comparative) sous la forme d'émulsions de type hui le-dans-eau.

| Composition | C1 | C2 |
|---|---|---|
| • Mélange d'alcool cétylstéarylique et d'alcool cétylstéarylique oxyéthyléné (33 OE) 80/20 SINNOWAW AO -HENKEL) | 7 | 7 |
| • Mélange de mono et distéarate de glycérol (CERASYNT SD-V ISP) | 2 | 2 |
| • Alcool cétylique | 1,5 | 1,5 |
| • Polydiméthyl siloxane (DOW CORNING 200 FLUID -DOW CORNING) | 1,5 | 1,5 |
| • Benzoate d'alcools en C12/C15 (WITCONOL TN-WITCO) | | 15 |
| • Lauroyl sarcosinate d'isopropyle (ELDEW SL205-AJINOMOTO) | 15 | |
| • Uvinul T150 | 10 | 10 |
| • Glycérine | 20 | 20 |
| • Conservateurs | qs | qs |
| • Eau déminéralisée qsp | 100 g | 100 g |

.Pour chacune des formulations ainsi préparées, on a ensuite déterminé le facteur de protection solaire (FPS) qui leur était attaché. Celui-ci a été déterminé en utilisant la méthode *in vitro* décrite par B.L. DIFFEY et al. dans J. Soc. Cosmet. Chem. 40-127-133 (1989), cette méthode consiste à déterminer les facteurs de protection monochromatiques tous les 5 nm dans une gamme de longueurs d'onde de 290 à 400 nm et à calculer à partir de ceux-ci le facteur de protection solaire selon une équation mathématique donnée.

Les résultats obtenus ont été les suivants :

| **Formules** | **Huile** | **FPS in vitro** |
|---|---|---|
| C1 | Ester N-acylé d'acide aminé | 10,1+0,4 |
| C2 comparatif | Miglyol 212 | 7,9+0,7 |

Ces résultats montrent clairement l'amélioration du facteur de protection solaire de la composition selon l'invention due à la présence d'un ester N-acylé d'acide aminé.

## Revendications

1. Composition **caractérisée par le fait qu'**elle comprend,
(i) au moins un dérivé de 1,3,5-triazine, et
(ii) au moins un ester choisi parmi les esters N-acylés d'acide aminé de formule
R'₁(CO)N(R'₂)CH(R'₃)(CH₂)ₙ(CO)OR'₄
dans laquelle :
n est un entier égal à 0,1 ou 2,
R'₁ représente un radical alkyle ou alcényle en C₅ à C₂₁, linéaire ou ramifié,
R'₂ représente un atome d'hydrogène ou un groupe alkyle en C₁ à C₃,
R'₃ représente un radical choisi dans le groupe formé par un atome d'hydrogène, un groupe méthyle, un groupe éthyle, une chaîne alkyle linéaire ou ramifiée en C₃ ou C₄,
R'₄ représente un radical alkyle en C₁ à C₁₀ linéaire ou ramifié, ou un radical alcényle en C₂ à C₁₀ linéaire ou ramifié ou un reste stérol.

2. Composition selon la revendication 1, **caractérisée en ce que** le dérivé de 1,3,5-triazine répond à la formule (I) suivante : 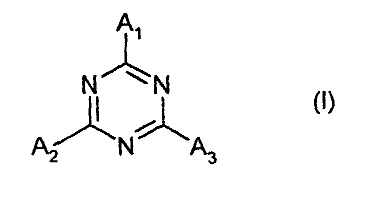 dans laquelle les radicaux A₁, A₂ et A₃, identiques ou différents sont choisis parmi les groupes de formules (II) à (IX) suivantes : 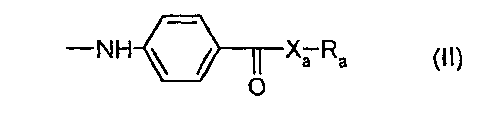 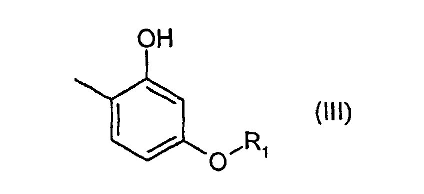 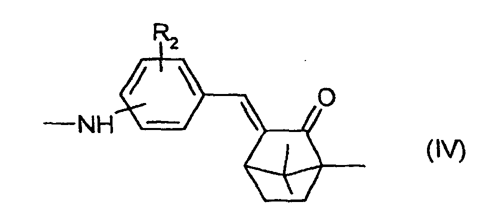 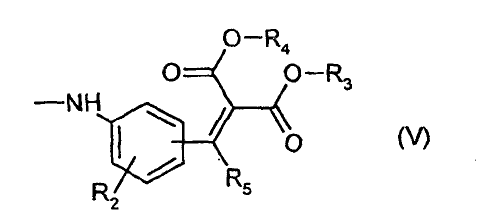 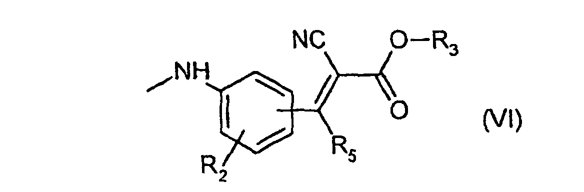 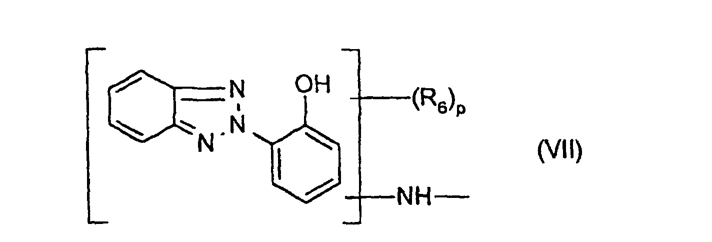 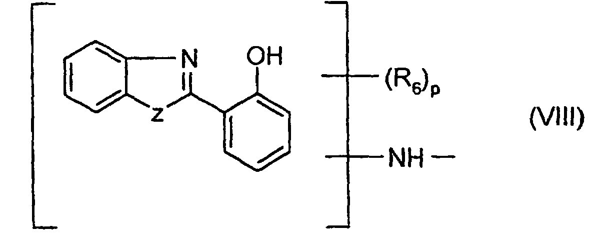 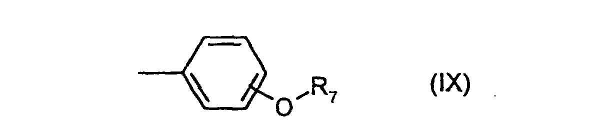 dans laquelle :
- Xₐ représente l'oxygène ou -NH-;
- Rₐ est choisi parmi l'hydrogène; un métal alcalin; un radical ammonium éventuellement substitué par un ou plusieurs radicaux alkyles linéaire ou ramifié en C₁-C₁₈ ou hydroxyalkyles linéaire ou ramifié en C₁-C₁₈; un radical alkyle linéaire ou ramifié en C₁-C₁₈, de préférence en C₆-C₁₂; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé ; un radical de formule (X), (XI) ou (XII) suivantes : 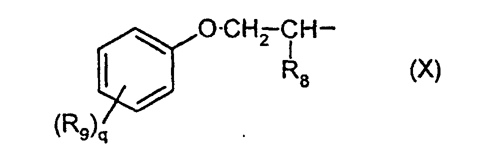 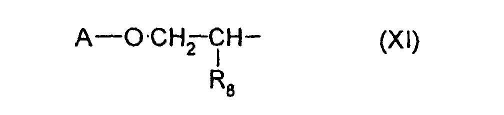 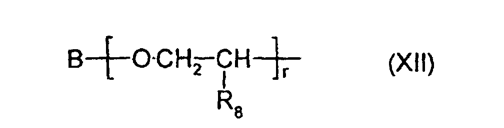
dans lesquelles :
- R₈ est l'hydrogène ou un radical méthyle;
- R₉ est un radical alkyle en C₁-C₉;
- q est un nombre entier égal à 0; 1; 2; 3;
- r est un nombre entier égal à 1; 2; 3; 4; 5; 6; 7; 8; 9; 10;
- A est un radical alkyle en C₄-C₈ ou un radical cycloalkyle en C₅-C₈ ;
- B est choisi parmi : un radical alkyle linéaire ou ramifié en C₁-C₈; un radical cycloalkyle en C₅-C₈; un radical aryle éventuellement substitué par un ou plusieurs radicaux alkyles en C₁-C₄.
- R₁ désigne un radical alkyle en C₃-C₁₈ ; un radical alcényle en C₂-C₁₈ ; un reste de formule -CH₂-CH(OH)-CH₂-OT₁ où T₁ est un atome d'hydrogène ou un radical alkyle en C₁-C₈ ; un reste de formule (XIII) suivante : 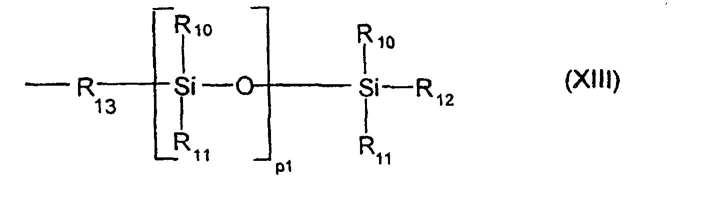
dans laquelle :
- R₁₃ désigne une liaison covalente ; un radical alkyle linéaire ou ramifié en C₁-C₄ ou bien un radical de formule -Cₘ₁H₂ₘ₁- ou-Cₘ₁H₂ₘ₁-O- où m₁ est un entier égal à 1; 2; 3; 4 ;
- p₁ est un entier égal à 0; 1; 2; 3; 4; 5 ;
- les radicaux R₁₀, R₁₁ et R₁₂, identiques ou différents, désignent un radical alkyle en C₁-C₁₈ ; un radical alcoxy en C₁-C₁₈ ou un radical de formule : 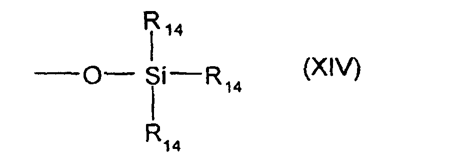
où R₁₄ est un radical alkyle en C₁-C₅.
- R₂ désigne un atome d'hydrogène, un radical alkyle en C₁-C₄ linéaire ou ramifié ou un radical alkoxy en C₁-C₄ ;
- R₃ et R₄, identiques ou différents désignent un radical alkyle linéaire ou ramifié en C₁-C₂₀ ;
- R₅ représente un atome d'hydrogène ou un radical phényle éventuellement substitué par un halogène ou par un radical alkyle en C₁-C₄ ou par un radical alcoxy en C₁-C₄ ;
- R₆ est un radical alkyle en C₁-C₈ linéaire ou ramifié, alkoxy en C₁-C₃ étant entendu que, dans ce dernier cas, deux R₆ adjacents d'un même noyau aromatique peuvent former ensemble un groupement alkylidène dioxy dans lequel le groupe alkylidène contient de 1 à 2 atomes de carbone, OH, NHCOCH₃ ou NH₂,
- R₇ désigne un atome d'hydrogène, un radical alkyle en C₁-C₁₀, un radical de formule : -(CH₂CHR₅-O)ₙ₁R₈ où n1 est un nombre de 1 à 16, ou bien un radical de structure -CH₂-CH-(OH)-CH₂OT₁ avec R₈ et T₁ ayant la même signification indiquée ci-dessus.
- Z représente l'oxygène, le soufre, -NH- ou -NR₃- avec R₃ représentant un radical alkyle en C₁-C₂₀ linéaire ou ramifié;
- p est 0, 1, 2 ou 3,
A₁ peut également être un halogène, un radical -N(R₃)₂, les deux R₃ pouvant former ensemble un cycle de 4 ou 5 atomes de carbone, ou un groupe -OR₃, R₃ ayant la même définition qu'au dessus.

3. Composition selon la revendication 2, **caractérisée par le fait que** le dérivé de 1,3,5-triazine répond à la formule (I) dans laquelle les A₁, A₂ et A₃ sont de formule (II) et présentant l'ensemble des caractéristiques suivantes :
- l'un des groupements Xₐ-Rₐ représente le radical -NH-Rₐ avec Rₐ choisi parmi : un radical cycloalkyle en C₅-C₁₂ éventuellement substitué par un ou plusieurs radicaux alkyles en C₁-C₄; un radical de formule (X), (XI) ou (XII) ci-dessus dans lesquelles :
- B est un radical alkyle en C₁-C₄ ;
- R₉ est le radical méthyle ;
- les 2 autres Xₐ-Rₐ représentent le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (X), (XI) ou (XII) ci-dessus dans lesquelles :
- B est un radical alkyle en C₁-C₄;
- R₉ est le radical méthyle.

4. Composition selon la revendication 2 telle que la 1,3,5-triazine répond à la formule (I) dans laquelle les A₁, A₂ et A₃ sont de formule (II) et présentant l'ensemble des caractéristiques suivantes :
- un ou deux Xₐ-Rₐ représente le radical -NH-Rₐ , avec Rₐ choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄; un radical de formule (X), (XI) ou (XII) ci-dessus dans lesquelles :
- B est un radical alkyle en C₁-C₄ ;
- R₉ est le radical méthyle ;
le ou les deux autres Xₐ-Rₐ étant le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (X), (XI) ou (XII) ci-dessus dans lesquelles :
- B est un radical alkyle en C₁-C₄;
- R₉ est le radical méthyle.

5. Composition selon la revendication 4 telle que la 1,3,5-triazine est la 2-[(p-(tertiobutylamido)anilino]-4,6-bis-[(p-(2'-éthylhexyl-l'-oxycarbonyl)anilino]-1,3,5-triazine.

6. Composition selon la revendication 2, telle que la 1,3,5-triazine répond à la formule (I) dans laquelle les A₁, A₂ et A₃ sont de formule (II) et présentant l'ensemble des caractéristiques suivantes :
- Xₐ sont identiques et représentent l'oxygène;
- Rₐ, identiques ou différents et représentent un radical alkyle en C₆-C₁₂ ou un radical polyoxyéthyléné comprenant de 1 à 6 unités d'oxyde d'éthylène et dont le groupe OH terminal est méthylé.

7. Composition selon la revendication 6 telle que la 1,3,5-triazine est la 2,4,6-tris[p-(2'-éthylhexyl-1'-oxycarbonyl)anilino]-1,3,5-triazine.

8. Composition selon la revendication 2 telle que la 1,3,5-triazine répond à la formule (I) dans laquelle les A₁ et A₂ sont de formule (III) et A₃ est de formule (IX) et présentant l'ensemble des caractéristiques suivantes : R₁, identiques ou différents, désignent un radical alkyle en C₃-C₁₈ ; un radical alcényle en C₂-C₁₈ ou bien un reste de formule -CH₂-CH(OH)-CH₂-OT₁ où T₁ est un atome d'hydrogène ou un radical alkyle en C₁-C₈ ; R₇ désigne un atome d'hydrogène, un radical alkyle en C₁-C₁₀.

9. Composition selon la revendication 8 telle que la 1,3,5-triazine est la 2,4-bis {[4-2-éthyl-hexyloxy)]-2-hydroxy]-phenyl}-6-(4-méthoxy-phenyl)-1,3,5-triazine.

10. Composition selon la revendication 2 telle que la 1,3,5-triazine répond à la formule (I) dans laquelle les A₁, A₂ et A₃ sont de formules (VII) à (XI).

11. Composition selon la revendication 10 telle que la 1,3,5-triazine est choisie dans le groupe formé par
- la 2,4,6-tris-(4'-amino benzalmalonate de diisobutyle)-s-triazine,
- la 2,4,6-tris-(4'-amino benzalmalonate de di(2-éthylhexyle))-s-triazine,
- la 2,4,6-tris-(4'-amino benzalmalonate de di(2-éthylhexyle))-6-chloro-s-triazine,
- la 2,4,6-tris-(4'-amino benzalmalonate de di(2-éthylhexyle))-6-(4'-aminobenzoate de 2-éthylhexyle)-s-triazine,
- la 2,4,6-tris-(4'-amino benzalmalonate de diisobutyle)-6-butoxy-s-triazine,
- la 2,4,6-tris-(4'-amino benzalmalonate de diisobutyle)-6-(2-éthylhexylamino)-s-triazine,
- la 2,4-bis-(4'-aminobenzylidène camphre)-6-(2-éthylhexylamino)-s-triazine,
- la 2,4-bis-(4'-aminobenzylidène camphre)-6-(4'-amino benzalmalonate de diisobutyle)-s-triazine,
- la 2,4,6-tris(4'-amino benzalmalonate de diéthyle)-s-triazine,
- la 2,4,6-tris(4'-amino benzalmalonate de diisopropyle)-s-triazine,
- la 2,4,6-tris (4'-amino benzalmalonate de diméthyle)-s-triazine,
- la 2,4,6-tris(α-cyano-4-aminocinnamate d'éthyle)-s-triazine.
- la 2,4,6-tris[(3'-benzotriazol-2-yl-2'-hydroxy-5'-methyl) phenylamino]-s-triazine,
- 2,4,6-tris[(3'-benzotriazol-2-yl-2'-hydroxy-5'-ter-octyl) phénylamino]-s-triazine.

12. Composition selon l'une des revendications précédentes **caractérisée en ce que** l'ester d'acide aminé et le N-lauroylsarcosinate d'isopropyle
CH₃-(CH₂)₁₀CO-N(CH₃)-CH₂-COO- CH-(CH₃)₂.

13. Composition selon l'une des revendications précédentes, **caractérisée en ce qu'**elle comprend dans un milieu physiologiquement acceptable de 0,05 à 15%, de préférence de 0,1 à 10 % de dérivé de 1,3,5-triazine en poids par rapport au poids total de la composition.

14. Composition selon l'une des revendications précédentes, **caractérisée en ce qu'**elle comprend dans un milieu physiologiquement acceptable de 0,1 à 50 %, de préférence de 1 à 30 % de dérivé d'ester N-acylés d'acide aminé en poids par rapport au poids total de la composition.

15. Composition selon l'une des revendications précédentes, **caractérisée en ce qu'**il s'agit d'une composition cosmétique qui contient outre le dérivé 1,3,5 triazine au moins un autre filtre organique, complémentaire, actif dans l'UV-A et/ou l'UV-B.

16. Composition selon la revendication 15, **caractérisée en ce que** le ou les filtres UV organiques sont choisis parmi les dérivés du dibenzoylméthane, les dérivés cinnamiques, les anthranilates ; les dérivés salicyliques, les dérivés du camphre ; les dérivés de la benzophénone ; les dérivés de β,β-diphénylacrylate, les dérivés de benzotriazole ; les dérivés de benzalmalonate ; les dérivés de benzimidazole ; les imadazolines ; les dérivés bis-benzoazolyle ; les dérivés de l'acide p-aminobenzoïque (PABA) ; les dérivés de méthylène bis-hydroxyphényl benzotriazole, les polymères filtres et silicones filtres, ; les dimères dérivés d'α-alkylstyrène et les 4,4-diarylbutadiènes.

17. Composition selon la revendication 16, **caractérisée par le fait que** le ou les filtres UV organiques sont choisis parmi les composés suivants :
- Ethylhexyl Salicylate,
- Octocrylene,
- Phenylbenzimidazole Sulfonic Acid,
- 4-Methylbenzylidene camphor,
- Benzimidazilate,
- Terephthalylidene Dicamphor Sulfonic,.
- Benzophenone-3,
- Benzophenone-4,
- Benzophenone-5,
- Methylene bis-Benzotriazolyl Tetramethylbutylphenol,
- Drometrizole Trisiloxane
- et leurs mélanges.

18. Composition selon l'une des revendications précédentes, **caractérisée en ce qu'**elle comprend en outre des nacres, des pigments ou des nanopigments d'oxydes métalliques, enrobés ou non.

19. Composition selon la revendication 18, **caractérisée en ce que** lesdits pigments ou nanopigments sont choisis parmi les oxydes de titane, de zinc, de fer, de zirconium, de cérium et leurs mélanges, enrobés ou non.

20. Composition selon l'une des revendications précédentes, **caractérisée en ce qu'**elle comprend en outre au moins un adjuvant choisi parmi les corps gras, les solvants organiques, les émulsionnants, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les antiinflammatoires, les antagonistes de substance P, les charges, les polymères, les propulseurs, les agents alcalinisants ou acidifiants, les colorants.

21. Composition selon l'une des revendications précédentes, **caractérisée en ce qu'**il s'agit d'une composition protectrice de l'épiderme humain ou d'une composition antisolaire et qu'elle se présente sous forme, d'une dispersion vésiculaire non ionique, d'une émulsion, en particulier d'une émulsion de type eau-dans huile, de type huile-dans-eau, d'une crème, ou d'une émulsion triple (E/H/E ou H/E/H), d'un lait, d'un gel, d'un gel crème, d'une suspension, d'une dispersion, d'une poudre, d'un bâtonnet solide, d'une mousse ou d'un spray.

22. Composition selon l'une des revendications 1 à 20, **caractérisée en ce qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

23. Composition selon l'une des revendications 1 à 20, **caractérisée en ce qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

24. Utilisation d'une composition selon l'une des revendications précédentes dans ou pour la fabrication de compositions cosmétiques ou dermatologiques destinées à la protection de la peau et/ou des lèvres et/ou des phanères contre le rayonnement ultraviolet, en particulier le rayonnement solaire.

25. Procédé de traitement cosmétique **caractérisé en ce qu'**il consiste à appliquer sur la peau et/ou les lèvres et/ou les phanères une composition selon l'une des revendications 1 à 21.

26. Utilisation d'au moins un ester N-acylés d'acide aminé de formule
R'₁(CO)N(R'₂)CH(R'₃)(CH₂)ₙ(CO)OR'₄
dans laquelle :
n est un entier égal à 0,1 ou 2,
R'₁ représente un radical alkyle ou alcényle en C₅ à C₂₁, linéaire ou ramifié,
R'₂ représente un atome d'hydrogène ou un groupe alkyle en C₁ à C₃,
R'₃ représente un radical choisi dans le groupe formé par un atome d'hydrogène, un groupe méthyle, un groupe éthyle, une chaîne alkyle linéaire ou ramifiée en C₃ ou C₄,
R'₄ représente un radical alkyle en C₁ à C₁₀ linéaire ou ramifié, ou un radical alcényle en C₂ à C₁₀ linéaire ou ramifié ou un reste stérol dans des compositions anti-solaires contenant un dérivé de 1,3,5-triazine en vue d'améliorer le facteur de protection solaire de cette composition.

## Patentansprüche

1. Zusammensetzung, **dadurch gekennzeichnet, dass** sie enthält:
(i) mindestens ein 1,3,5-Triazinderivat, und
(ii) mindestens einen Ester, der unter den N-acylierten Aminosäureestern der folgenden Formel ausgewählt ist:
R'₁(CO)N(R'₂)CH(R'₃)(CH₂)ₙ(CO)OR'₄
worin bedeuten:
n 0, 1 oder 2,
R'₁ eine geradkettige oder verzweigte Alkyl- oder Alkenylgruppe mit 5 bis 21 Kohlenstoffatomen,
R'₂ ein Wasserstoffatom oder eine C₁₋₃-Alkylgruppe,
R'₃ eine Gruppe, die unter einem Halogenatom, einer Methylgruppe, einer Ethylgruppe oder einer geradkettigen oder verzweigten Alkylgruppe mit 3 oder 4 Kohlenstoffatomen ausgewählt ist, und
R'₄ eine geradkettige oder verzweigte C₁₋₁₀-Alkylgruppe, eine geradkettige oder verzweigte C₂₋₁₀-Alkenylgruppe oder einen Sterinrest.

2. Zusammensetzung nach Anspruch 1, **dadurch gekennzeichnet, dass** das 1,3,5-Triazinderivat der folgenden Formel (I) entspricht: 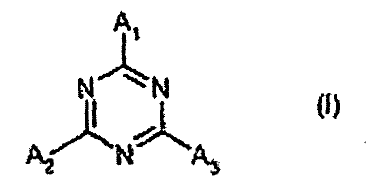 worin die Gruppen A₁, A₂ und A₃, die gleich oder voneinander verschieden sind, unter den Gruppen der folgenden Formeln (II) bis (IX) ausgewählt sind: 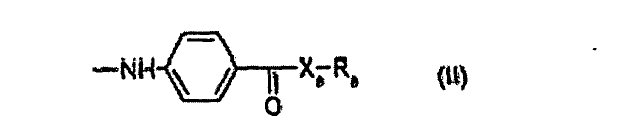 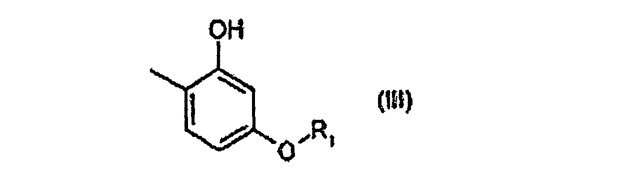 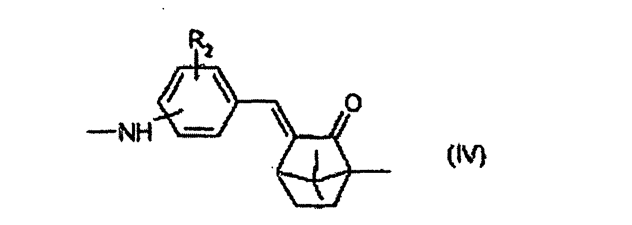 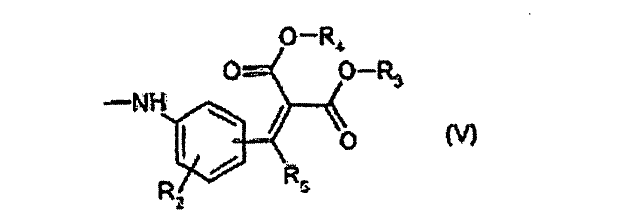 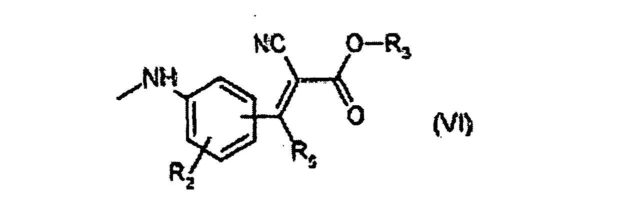 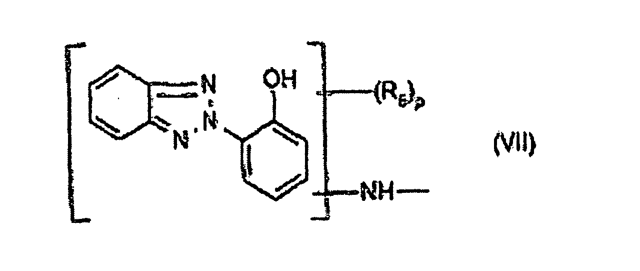 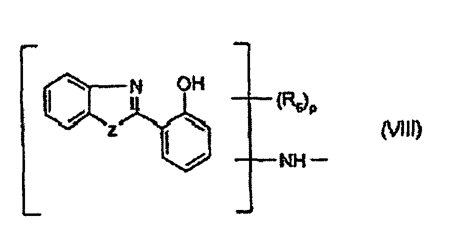 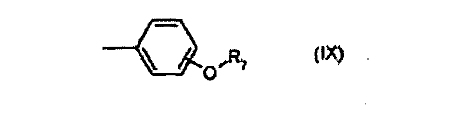 worin bedeuten:
- Xₐ bedeutet Sauerstoff oder -NH-;
- Rₐ ist ausgewählt unter Wasserstoff; einem Alkalimetall; einer Ammoniumgruppe, die gegebenenfalls mit einer oder mehreren geradkettigen oder verzweigten C₁₋₁₈-Alkylgruppen oder geradkettigen oder verzweigten C₁₋₁₈-Hydroxyalkylgruppen substituiert ist; geradkettigen oder verzweigten C₁₋₁₈-Alkylgruppen und vorzugsweise C₆₋₁₂-Alkylgruppen; C₅₋₁₂-Cycloalkylgruppen, die gegebenenfalls mit einer oder mehreren C₁₋₄-Alkylgruppen substituiert sind; polyethoxylierten Gruppen, die 1 bis 6 Ethylenoxideinheiten enthalten und deren endständige OH-Gruppe methyliert ist; Gruppen der folgenden Formeln (X), (XI) oder (XII): 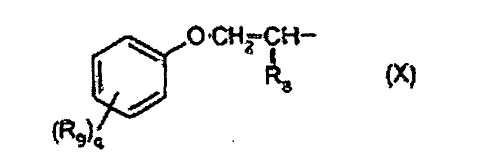 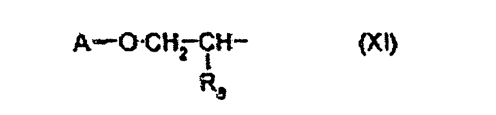 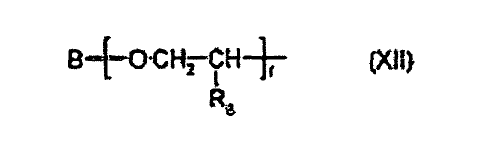
worin bedeuten:
- R₈ Wasserstoff oder Methyl;
- R₉ C₁₋₉-Alkyl;
- q 0, 1, 2 oder 3;
- r eine ganze Zahl gleich 1, 2, 3, 4, 5, 6, 7, 8, 9 oder 10;
- A C₄₋₈-Alkyl oder C₅₋₈-Cycloalkyl;
- B eine geradkettige oder verzweigte C₁₋₈-Alkylgruppe; eine C₅₋₈-Cycloalkylgruppe; eine Arylgruppe, die gegebenenfalls mit einer oder mehreren C₁₋₄-Alkylgruppen substituiert ist;
- R₁ C₃₋₁₈-Alkyl; C₂₋₁₈-Alkenyl; einen Rest der Formel -CH₂CH(OH)-CH₂-OT₁, wobei T₁ Wasserstoff oder C₁₋₈-Alkyl bedeutet; einen Rest der folgenden Formel (XIII): 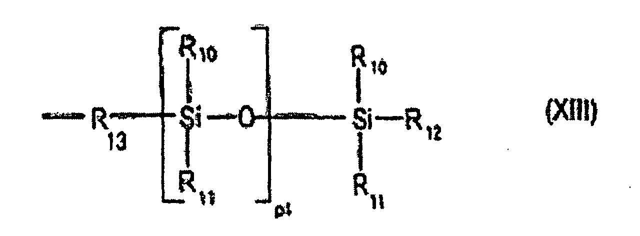
worin bedeuten:
- R₁₃ eine kovalente Bindung; eine geradkettige oder verzweigte C₁₋₄-Alkylgruppe oder eine Gruppe der Formel -Cₘ₁H₂ₘ₁- oder -Cₘ₁H₂ₘ₁-O-, worin m₁ eine ganze Zahl gleich 1, 2, 3 oder 4 bedeutet;
- p₁ eine ganze Zahl gleich 1, 2, 3, 4 oder 5;
- die Gruppen R₁₀, R₁₁ und R₁₂, die identisch oder voneinander verschieden sind, C₁₋₁₈-Alkyl; C₁₋₁₈-Alkoxy oder eine Gruppe der Formel 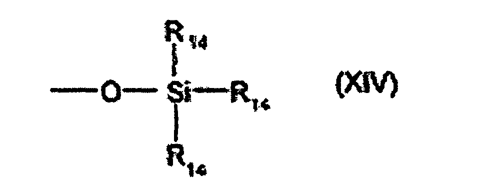
wobei R₁₄ C₁₋₅-Alkyl bedeutet;
- R₂ Wasserstoff, eine geradkettige oder verzweigte C₁₋₄-Alkylgruppe oder eine C₁₋₄-Alkoxygruppe;
- R₃ und R₄, die gleich oder verschieden sind, eine geradkettige oder verzweigte C₁₋₂₀-Alkylgruppe;
- Rs ein Wasserstoffatom oder eine Phenylgruppe, die gegebenenfalls mit einem Halogen oder einer C₁₋₄-Alkylgruppe oder einer C₁₋₄-Alkoxygruppe substituiert ist;
- R₆ eine geradkettige oder verzweigte C₁₋₈-Alkylgruppe, eine C₁₋₃-Alkoxygruppe, mit der Maßgabe, dass im letztgenannten Fall zwei Gruppen R₆, die an einem aromatischen Kern benachbart sind, gemeinsam eine Alkylidendioxygruppe bilden können, worin die Alkylidengruppe 1 bis 2 Kohlenstoffatome enthält, OH, NHCOCH₃ oder NH₂,
- R₇ ein Wasserstoffatom, C₁₋₁₀-Alkyl, eine Gruppe der Formel -(CH₂CHR₅-O)ₙ₁R₈, wobei n₁ eine Zahl von 1 bis 16 bedeutet, oder eine Gruppe der Struktur -CH₂CH-(OH)-CH₂OT₁, wobei R₈ und T₁ die oben angegebenen Bedeutungen aufweisen;
- Z Sauerstoff, Schwefel, -NH-, oder -NR₃, wobei R₃ eine geradkettige oder verzweigte C₁₋₂₀-Alkylgruppe bedeutet;
- p bedeutet 0, 1, 2 oder 3,
wobei A₁ auch ein Halogen, eine Gruppe -N(R₃)₂, wobei die beiden Gruppen R₃ gemeinsam einen Ring mit 4 oder 5 Kohlenstoffatomen bilden können, oder eine Gruppe -OR₃ bedeuten kann, wobei R₃ die oben angegebene Bedeutung aufweist.

3. Zusammensetzung nach Anspruch 2, **dadurch gekennzeichnet, dass** das 1,3,5-Triazinderivat der Formel (I) entspricht, worin die Gruppen A₁, A₂ und A₃ der Formel (II) entsprechen und alle folgenden Eigenschaften aufweisen:
- eine der Gruppen Xₐ-Rₐ bedeutet die Gruppe -NH-Rₐ, wobei Rₐ ausgewählt ist unter: einer C₅₋₁₂-Cycloalkylgruppe, die gegebenenfalls mit einer oder mehreren C₁₋₄-Alkylgruppen substituiert ist; einer Gruppe der Formel (X), (XI) oder (XII), worin bedeuten:
- B eine C₁₋₄-Alkylgruppe;
- R₉ die Methylgruppe;
- die beiden anderen Gruppen Xₐ-Rₐ bedeuten die Gruppe -O-Rₐ, wobei die Gruppen Rₐ, die gleich oder verschieden sind, ausgewählt sind unter: Wasserstoff; einem Alkalimetall; einer Ammoniumgruppe, die gegebenenfalls mit einer oder mehreren Alkyl- oder Hydroxyalkylgruppen substituiert ist; einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer Gruppe der oben angegebenen Formel (X), (XI) oder (XII), worin bedeuten:
- B C₁₋₄-Alkyl;
- R₉ Methyl.

4. Zusammensetzung nach Anspruch 2, wobei das 1,3,5-Triazin der Formel (I) entspricht, worin die Gruppen A₁, A₂ und A₃ der Formel (II) entsprechen und die gesamten folgenden Eigenschaften aufweisen:
- eine oder zwei Gruppen Xₐ-Rₐ bedeuten die Gruppe -NH-Rₐ, wobei Rₐ ausgewählt ist unter: einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer Gruppe der oben angegebenen Formel (X), (XI) oder (XII), worin bedeuten:
- B C₁₋₄-Alkyl;
- R₉ Methyl;
wobei die andere Gruppe Xₐ-Rₐ oder die beiden anderen Gruppen Xₐ-Rₐ die Gruppe -O-Rₐ bedeuten, worin die Gruppen Rₐ, die gleich oder verschieden sind, ausgewählt sind unter: Wasserstoff; einem Alkalimetall; einer Ammoniumgruppe, die gegebenenfalls mit einer oder mehreren Alkyl- oder Hydroxyalkylgruppen substituiert ist; einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer Gruppe der oben angegebenen Formel (X), (XI) oder (XII), worin bedeuten:
- B C₁₋₄-Alkyl;
- R₉ Methyl.

5. Zusammensetzung nach Anspruch 4, wobei das 1,3,5-Triazin das 2-[(p-(*t*-Butylamido)anilino]-4,6-bis-[(p-(2'-ethylhexyl-1'-oxycarbonyl)anilino]-1,3,5-triazin ist.

6. Zusammensetzung nach Anspruch 2, wobei das 1,3,5-Triazin der Formel (I) entspricht, worin die Gruppen A₁, A₂ und A₃ der Formel (II) entsprechen und die gesamten folgenden Eigenschaften aufweisen:
- die Gruppen Xₐ sind identisch und bedeuten Sauerstoff;
- die Gruppen Rₐ, die gleich oder verschieden sind, bedeuten eine C₆₋₁₂-Alkylgruppe oder eine polyethoxylierte Gruppe, die 1 bis 6 Ethylenoxideinheiten enthält und deren endständige OH-Gruppe methyliert ist.

7. Zusammensetzung nach Anspruch 6, wobei das 1,3,5-Triazin das 2,4,6-Tris[p-(2'-Ethylhexyl-1'-oxycarbonyl)anilino]-1,3,5-triazin ist.

8. Zusammensetzung nach Anspruch 2, wobei das 1,3,5-Triazin der Formel (I) entspricht, worin die Gruppen A₁ und A₂ der Formel (III) entsprechen und die Gruppe A₃ der Formel (IX) entspricht, und die gesamten folgenden Eigenschaften aufweist: die Gruppen R₁, die gleich oder verschieden sind, bedeuten eine C₃₋₁₈-Alkylgruppe; eine C₂₋₁₈-Alkenylgruppe oder einen Rest der Formel -CH₂-CH(OH)-CH₂-OT₁, wobei T₁ ein Wasserstoffatom oder eine C₁₋₈-Alkylgruppe bedeutet; R₇ bedeutet ein Wasserstoffatom oder eine C₁₋₁₀-Alkylgruppe.

9. Zusammensetzung nach Anspruch 8, wobei das 1,3,5-Triazin das 2,4-Bis-{[4-2-ethyl-hexyloxy)]-2-hydroxy]-phenyl}-6-(4-methoxy-phenyl)-1,3,5-triazin ist.

10. Zusammensetzung nach Anspruch 2, wobei das 1,3,5-Triazin der Formel (I) entspricht, worin die Gruppen A₁, A₂ und A₃ den Formeln (VII) bis (XI) entsprechen.

11. Zusammensetzung nach Anspruch 10, wobei das 1,3,5-Triazin unter den folgenden Verbindungen ausgewählt ist:
- 2,4,6-Tris-(diisobutyl-4'-amino-benzalmalonat)-s-triazin,
- 2,4,6-Tris(di(2-ethylhexyl)-4'-amino-benzalmalonat)-s-triazin,
- 2,4,6-Tris(di(2-ethylhexyl)-4'-amino-benzalmalonat)-6-chlor-striazin,
- 2,4,6-Tris-(di(2-ethylhexyl)-4'-amino-benzalinalonat)-6-(2-ethylhexyl-4'-amino-benzoat)-s-triazin,
- 2,4,6-Tris-(diisobutyl-4'-amino-benzalmalonat)-6-butoxy-striazin;
- 2,4,6-Tris-(diisobutyl-4'-amino-benzalmalonat)-6-(2-ethylhexylamino)-s-triazin;
- 2,4-Bis-(4'-aminobenylidencampher)-6-(2-ethylhexylamino)-striazin;
- 2,4-Bis-(4'-aminobenzylidencampher)-6-(diisobutyl-4'-aminobenzalmalonat)-s-triazin;
- 2,4,6-Tris(diethyl-4'-amino-benzalmalonat)-s-triazin;
- 2,4,6-Tris(diisopropyl-4'-amino-benzalmalonat)-s-triazin;
- 2,4,6-Tris(dimethyl-4'-amino-benzalmalonat)-s-triazin;
- 2,4,6-Tris(ethyl-4-amino-α-cyano-cinnamat)-s-triazin;
- 2,4,6-Tris-[(3'-benzotriazol-2-yl-2'-hydroxy-5'-methyl)phenylamino]-s-triazin;
- 2,4,6-Tris[(3'-benzotriazol-2-yl-2'-hydroxy-5'-t-octyl)-phenylamino]-s-triazin.

12. Zusammensetzung nach einem der vorhergehenden Ansprüche, **dadurch gekennzeichnet, dass** der Aminosäureester das Isopropyl-N-lauroylsarcosinat ist
CH₃-(CH₂)₁₀CO-N(CH₃)-CH₂-COO-CH-(CH₃)₂.

13. Zusammensetzung nach einem der vorhergehenden Ansprüche, **dadurch gekennzeichnet, dass** sie in einem physiologisch akzeptablen Medium 0,05 bis 15 Gew.-% und vorzugsweise 0,1 bis 10 Gew.-% 1,3,5-Triazinderivat, bezogen auf das Gesamtgewicht der Zusammensetzung, enthält.

14. Zusammensetzung nach einem der vorhergehenden Ansprüche, **dadurch gekennzeichnet, dass** sie in einem physiologischen akzeptablen Medium 0,1 bis 50 Gew.-% und vorzugsweise 1 bis 30 Gew.-% des N-acylierten Aminosäureesterderivats, bezogen auf das Gesamtgewicht der Zusammensetzung, enthält.

15. Zusammensetzung nach einem der vorhergehenden Ansprüche, **dadurch gekennzeichnet, dass** es sich um eine kosmetische Zusammensetzung handelt, die neben dem 1,3,5-Triazinderivat mindestens ein weiteres, ergänzendes, im UV-A- und/oder UV-B-Bereich wirksames, organisches Filter enthält.

16. Zusammensetzung nach Anspruch 15, **dadurch gekennzeichnet, dass** das oder die organischen UV-Filter ausgewählt sind unter: Dibenzoylmethanderivaten, Zimtsäurederivaten, Anthranilaten; Salicylsäurederivaten, Campherderivaten, Benzophenonderivaten; β,β-Diphenylacrylatderivaten, Benzotriazolderivaten; Benzalmalonatderivaten; Benzimidazolderivaten; Imidazolinen; Bisbenzoaxozylderivaten; p-Aminobenzoesäureserivaten (PABA); Methylen-bis-hydroxyphenyl-benzotriazolderivaten, Filterpolymeren und Siliconfiltern; Dimeren, die von α-Alkylstyrol und 4,4-Diarylbutadienen abgeleitet sind.

17. Zusammensetzung nach Anspruch 16, **dadurch gekennzeichnet, dass** das oder die organischen UV-Filter unter den folgenden Verbindungen ausgewählt sind:
- Ethylhexyl Salicylate,
- Octocrylene,
- Phenylbenzimidazole Sulfonic Acid,
- 4-Methylbenzylidene camphor,
- Benzimidazilate,
- Terephthalylidene Dicamphor Sulfonic,
- Benzophenone-3,
- Benzophenone-4,
- Benzophenone-5,
- Methylene bis-Benzotriazolyl Tetramethylbutylphenol,
- Drometrizole Trisiloxane
- und deren Gemischen.

18. Zusammensetzung nach einem der vorhergehenden Ansprüche, **dadurch gekennzeichnet, dass** sie ferner Perlglanzpigmente, Pigmente oder Nanopigmente von Metalloxiden enthält, welche gegebenenfalls umhüllt sind.

19. Zusammensetzung nach Anspruch 18, **dadurch gekennzeichnet, dass** die Pigmente oder Nanopigmente unter den Oxiden von Titan, Zink, Eisen, Zirconium, Cer und deren Gemischen ausgewählt sind, wobei sie umhüllt oder nicht umhüllt vorliegen.

20. Zusammensetzung nach einem der vorhergehenden Ansprüche, **dadurch gekennzeichnet, dass** sie ferner mindestens einen Zusatzstoff enthält, der unter den Fettsubstanzen, organischen Lösungsmitteln, Emulgatoren, ionischen oder nichtionischen Verdickungsmitteln, beruhigenden Stoffen, Antioxidantien, Radikalfängem für freie Radikale, Trübungsmitteln, Stabilisatoren, Emollientien, Siliconen, α-Hydroxysäuren, Schaumverhütungsmitteln, Hydratisierungsmitteln, Vitaminen, Insekten abwehrenden Stoffen, Parfums, Konservierungsmitteln, grenzflächenaktiven Stoffen, Antiphlogistika, Substanz P-Antagonisten, Füllstoffen, Polymeren, Treibmitteln, Alkalisierungsmitteln oder Ansäuerungsmitteln und Farbmitteln ausgewählt ist.

21. Zusammensetzung nach einem der vorhergehenden Ansprüche, **dadurch gekennzeichnet, dass** es sich um eine Zusammensetzung zum Schutz der menschlichen Epidermis oder ein Sonnenschutzmittel handelt und dadurch, dass sie in Form einer nichtionischen Vesikeldispersion, einer Emulsion und insbesondere einer Emulsion vom Typ Wasser-in-Öl oder vom Typ Öl-in-Wasser, als Creme oder in Form einer dreifachen Emulsion (W/O/W oder O/W/O), als Milch, Gel, Gelcreme, Suspension, Dispersion, Pulver, fester Stift, Schaum oder Spray vorliegt.

22. Zusammensetzung nach einem der Ansprüche 1 bis 20, **dadurch gekennzeichnet, dass** es sich um eine Zusammensetzung zum Schminken der Wimpern, Augenbrauen oder der Haut handelt, und dadurch, dass sie in fester oder pastöser, wässriger oder wasserfreier Form, als Emulsion, Suspension oder Dispersion vorliegt.

23. Zusammensetzung nach einem der Ansprüche 1 bis 20, **dadurch gekennzeichnet, dass** es sich um eine Zusammensetzung handelt, die für den Schutz der Haare gegen UV-Strahlung vorgesehen ist, und dadurch, dass sie als Haarwaschmittel, Lotion, Gel, Emulsion oder nichtionische Vesikeldispersion vorliegt.

24. Verwendung einer Zusammensetzung nach einem der vorhergehenden Ansprüche in kosmetischen oder dermatologischen Zusammensetzungen, die für den Schutz der Haut und/oder der Lippen und/oder der Hautanhangsgebilde gegen UV-Strahlung und insbesondere gegenüber Sonnenlicht vorgesehen sind, oder für die Herstellung solcher Zusammensetzungen.

25. Verfahren zur kosmetischen Behandlung, **dadurch gekennzeichnet, dass** es darin besteht, auf die Haut und/oder die Lippen und/oder die Hautanhangsgebilde eine Zusammensetzung nach einem der Ansprüche 1 bis 21 aufzutragen.

26. Verwendung mindestens eines N-acylierten Aminosäureesters der folgenden Formel:
R'₁(CO)N(R'₂)CH(R'₃)(CH₂)ₙ(CO)OR'₄
worin bedeuten:
n 0, 1 oder 2,
R'₁ eine geradkettige oder verzweigte Alkyl- oder Alkenylgruppe mit 5 bis 21 Kohlenstoffatomen,
R'₂ ein Wasserstoffatom oder eine C₁₋₃-Alkylgruppe,
R'₃ eine Gruppe, die unter einem Halogenatom, Methyl, Ethyl oder einer geradkettigen oder verzweigten Alkylgruppe mit 3 oder 4 Kohlenstoffatomen ausgewählt ist, und
R'₄ eine geradkettige oder verzweigte C₁₋₁₀-Alkylgruppe, eine geradkettige oder verzweigte C₂₋₁₀-Alkenylgruppe oder einen Sterinrest,
in Sonnenschutzmitteln, die ein 1,3,5-Triazinderivat enthalten, zur Verbesserung des Lichtschutzfaktors dieser Zusammensetzung.

## Claims

1. Composition, **characterized in that** it comprises:
(i) at least one 1,3,5-triazine derivative, and
(ii) at least one ester chosen from N-acyl amino acid esters of formula
R'₁(CO)N(R'₂)CH(R'₃) (CH₂)ₙ(CO)OR'₄
in which:
n is an integer equal to 0, 1 or 2,
R'₁ represents a linear or branched C₅ to C₂₁ alkyl or alkenyl radical,
R'₂ represents a hydrogen atom or a C₁ to C₃ alkyl group,
R'₃ represents a radical chosen from the group formed by a hydrogen atom, a methyl group, an ethyl group and a linear or branched C₃ or C₄ alkyl chain,
R'₄ represents a linear or branched C₁ to C₁₀ alkyl radical or a linear or branched C₂ to C₁₀ alkenyl radical or a sterol residue.

2. Composition according to Claim 1, **characterized in that** the 1,3,5-triazine derivative corresponds to formula (I) below: 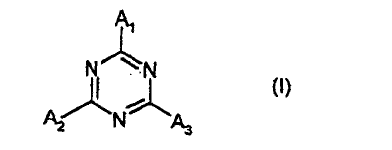 in which the radicals A₁, A₂ and A₃, which may be identical or different, are chosen from the groups of formulae (II) to (IX) below: 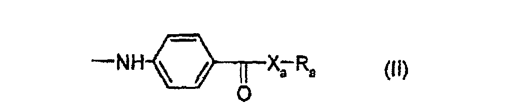 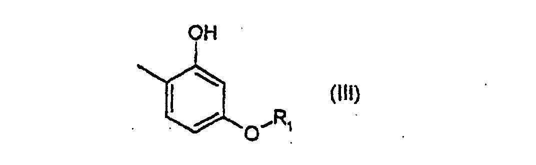 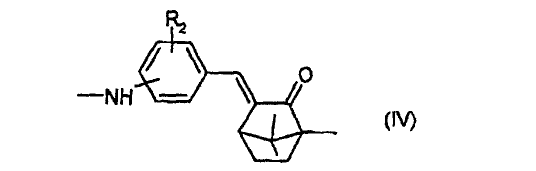 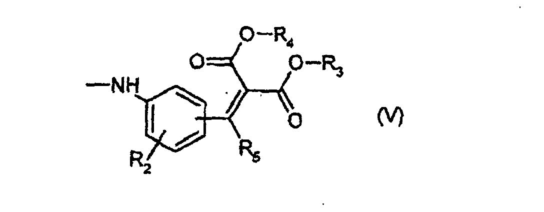 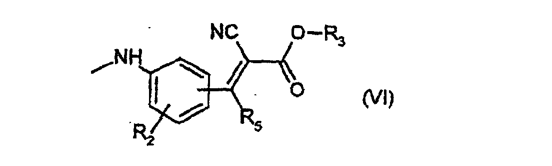 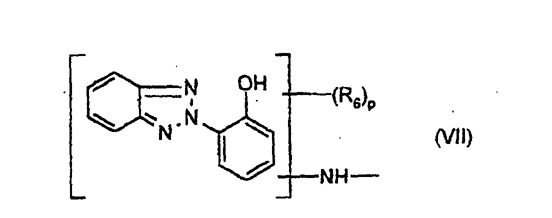 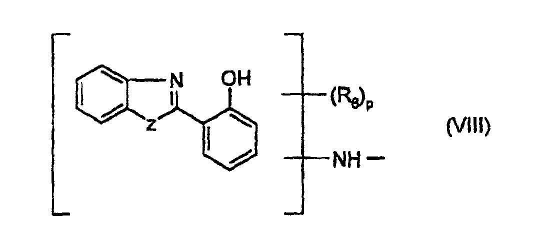 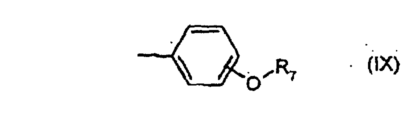 in which:
- Xₐ represents oxygen or -NH-;
- Rₐ is chosen from hydrogen; an alkali metal; an ammonium radical optionally substituted with one or more linear or branched C₁-C₁₈ alkyl or linear or branched C₁-C₁₈ hydroxyalkyl radicals; a linear or branched C₁-C₁₈ and preferably C₆-C₁₂ alkyl radical; a C₅-C₁₂ cycloalkyl radical optionally substituted with one or more C₁-C₄ alkyl radicals; a polyoxyethylenated radical comprising from 1 to 6 ethylene oxide units, the terminal OH group of which is methylated; a radical of formula (X), (XI) or (XII) below: 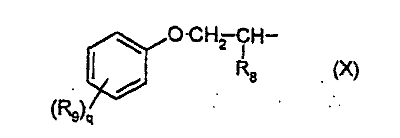 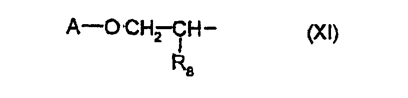 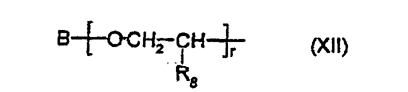
in which:
- R₈ is hydrogen or a methyl radical;
- R₉ is a C₁-C₉ alkyl radical;
- q is an integer equal to 0; 1; 2; 3;
- r is an integer equal to 1; 2; 3; 4; 5; 6; 7; 8; 9; 10;
- A is a C₄-C₈ alkyl radical or a C₅-C₈ cycloalkyl radical;
- B is chosen from: a linear or branched C₁-C₈ alkyl radical; a C₅-C₈ cycloalkyl radical; an aryl radical optionally substituted with one or more C₁-C₄ alkyl radicals.
- R₁ denotes a C₃-C₁₈ alkyl radical; a C₂-C₁₈ alkenyl radical; a residue of formula -CH₂-CH(OH)-CH₂-OT₁ in which T₁ is a hydrogen atom or a C₁-C₈ alkyl radical; a residue of formula (XIII) below: 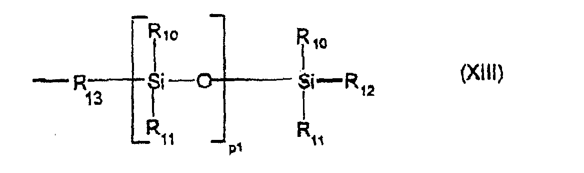
in which:
- R₁₃ denotes a covalent bond; a linear or branched C₁-C₄ alkyl radical or a radical of formula -Cₘ₁H₂ₘ₁- or -Cₘ₁H₂ₘ₁-O- in which m₁ is an integer equal to 1; 2; 3; 4;
- p₁ is an integer equal to 0; 1; 2; 3; 4; 5;
- the radicals R₁₀, R₁₁ and R₁₂, which may be identical or different, denote a C₁-C₁₈ alkyl radical; a C₁-C₁₈ alkoxy radical or a radical of formula: 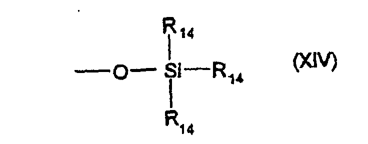
in which R₁₄ is a C₁-C₅ alkyl radical,
- R₂ denotes a hydrogen atom, a linear or branched C₁-₄ alkyl radical or a C₁-C₄ alkoxy radical;
- R₃ and R₄, which may be identical or different, denote a linear or branched C₁-C₂₀ alkyl radical;
- R₅ represents a hydrogen atom or a phenyl radical optionally substituted with a halogen or with a C₁-C₄ alkyl radical or with a C₁-C₄ alkoxy radical;
- R₆ is a linear or branched C₁-C₈ alkyl radical or a C₁-C₃ alkoxy radical, it being understood that, in the latter case, two adjacent radicals R₆ on the same aromatic nucleus can together form an alkylidenedioxy group in which the alkylidene group contains 1 or 2 carbon atoms, OH, NHCOCH₃ or NH₂,
- R₇ denotes a hydrogen atom, a C₁-C₁₀ alkyl radical, a radical of formula: - (CH₂CHR₅-O)ₙ₁R₈ in which n1 is a number from 1 to 16, or a radical of structure -CH₂-CH-(OH)-CH₂OT₁ with R₈ and T₁ having the same meaning as indicated above,
- Z represents oxygen, sulphur, -NH- or -NR₃- with R₃ representing a linear or branched C₁-C₂₀ alkyl radical;
- p is 0, 1, 2 or 3,
A₁ can also be a halogen, a radical -N(R₃)₂, the two radicals R₃ together possibly forming a ring of 4 or 5 carbon atoms, or a group -OR₃, R₃ having the same definition as above.

3. Composition according to Claim 2, **characterized in that** the 1,3,5-triazine derivative corresponds to formula (I) in which A₁, A₂ and A₃ are of formula (II) and have all of the following characteristics:
- one of the groups Xₐ-Rₐ represents a radical-NH-Rₐ with Rₐ chosen from: a C₅-C₁₂ cycloalkyl radical optionally substituted with one or more C₁-C₄ alkyl radicals; a radical of formula (X), (XI) or (XII) above in which:
- B is a C₁-C₄ alkyl radical;
- R₉ is a methyl radical;
- the other two groups Xₐ-Rₐ represent a radical -O-Rₐ with Rₐ, which may be identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (X), (XI) or (XII) above in which:
- B is a C₁-C₄ alkyl radical;
- R₉ is a methyl radical.

4. Composition according to Claim 2, such that the 1,3,5-triazine corresponds to formula (I) in which A₁, A₂ and A₃ are of formula (II) and have all of the following characteristics:
- one or two groups Xₐ-Rₐ represent a radical -NH-Rₐ, with Rₐ chosen from: a linear or branched C₁-C₁₈ alkyl radical; a C₅-C₁₂ cycloalkyl radical optionally substituted with one or more C₁-C₄ alkyl radicals; a radical of formula (X), (XI) or (XII) above in which:
- B is a C₁-C₄ alkyl radical;
- R₉ is a methyl radical;
the other or the other two group(s) Xₐ-Rₐ being a radical -O-Rₐ with Rₐ, which may be identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (X), (XI) or (XII) above in which:
- B is a C₁-C₄ alkyl radical;
- R₉ is a methyl radical.

5. Composition according to Claim 4, such that the 1,3,5-triazine is 2-[(p-(tert-butylamido)anilino]-4,6-bis[(p-(2'-ethylhexyl-1'-oxycarbonyl)anilino]-1,3,5-triazine.

6. Composition according to Claim 2, such that the 1,3,5-triazine corresponds to formula (I) in which A₁, A₂ and A₃ are of formula (II) and have all of the following characteristics:
- Xₐ are identical and represent oxygen;
- Rₐ, which may be identical or different, represent a C₆-C₁₂ alkyl radical or a polyoxyethylenated radical comprising from 1 to 6 ethylene oxide units and in which the terminal OH group is methylated.

7. Composition according to Claim 6, such that the 1,3,5-triazine is 2,4,6-tris[p-(2'-ethylhexyl-1'-oxycarbonyl)anilino]-1,3,5-triazine.

8. Composition according to Claim 2, such that the 1,3,5-triazine corresponds to formula (I) in which A₁ and A₂ are of formula (III) and A₃ is of formula (IX) and have all of the following characteristics: R₁, which may be identical or different, denote a C₃-C₁₈ alkyl radical; a C₂-C₁₈ alkenyl radical or a residue of formula -CH₂-CH(OH)-CH₂-OT₁ in which T₁ is a hydrogen atom or a C₁-C₈ alkyl radical; R₇ denotes a hydrogen atom or a C₁-C₁₀ alkyl radical.

9. Composition according to Claim 8, such that the 1,3,5-triazine is 2,4-bis{[4-2-ethylhexyloxy)]-2-hydroxy]phenyl}-6-(4-methoxyphenyl)-1,3,5-triazine.

10. Composition according to Claim 2, such that the 1,3,5-triazine corresponds to formula (I) in which A₁, A₂ and A₃ are of formulae (VII) to (XI).

11. Composition according to Claim 10, such that the 1,3,5-triazine is chosen from the group formed by:
- 2,4,6-tris(diisobutyl 4'-aminobenzalmalonate)-s-triazine,
- 2,4,6-tris(bis(2-ethylhexyl) 4'-aminobenzal-malonate)-s-triazine,
- 2,4,6-tris(bis(2-ethylhexyl) 4'-aminobenzal-malonate)-6-chloro-s-triazine,
- 2,4,6-tris(bis(2-ethylhexyl) 4'-aminobenzal-malonate)-6-(2-ethylhexyl 4'-aminobenzoate)-s-triazine,
- 2,4,6-tris(diisobutyl 4'-aminobenzalmalonate)-6-butoxy-s-triazine,
- 2,4,6-tris(diisobutyl 4'-aminobenzalmalonate)-6-(2-ethylhexylamino)-s-triazine,
- 2,4-bis(4'-aminobenzylidenecamphor)-6-(2-ethyl-hexylamino)-s-triazine,
- 2,4-bis(4'-aminobenzylidenecamphor)-6-(diisobutyl 4'-aminobenzalmalonate)-s-triazine,
- 2,4,6-tris(diethyl 4'-aminobenzalmalonate)-s-triazine,
- 2,4,6-tris(diisopropyl 4'-aminobenzalmalonate)-s-triazine,
- 2,4,6-tris(dimethyl 4'-aminobenzalmalonate)-s-triazine,
- 2,4,6-tris(ethyl α-cyano-4-aminocinnamate)-s-triazine,
- 2,4,6-tris[(3'-benzotriazol-2-yl-2'-hydroxy-5'-methyl)phenylamino]-s-triazine,
- 2,4,6-tris[(3'-benzotriazol-2-yl-2'-hydroxy-5'-tert-octyl)phenylamino]-s-triazine.

12. Composition according to one of the preceding claims, **characterized in that** the amino acid ester is isopropyl N-lauroylsarcosinate
CH₃- (CH₂)₁₀CO-N(CH₃)-CH₂-COO-CH- (CH₃)₂.

13. Composition according to one of the preceding claims, **characterized in that** it comprises, in a physiologically acceptable medium, from 0.05% to 15% and preferably from 0.1% to 10% of 1,3,5-triazine derivative by weight relative to the total weight of the composition.

14. Composition according to one of the preceding claims, **characterized in that** it comprises, in a physiologically acceptable medium, from 0.1% to 50% and preferably from 1% to 30% of N-acyl amino acid ester derivative by weight relative to the total weight of the composition.

15. Composition according to one of the preceding claims, **characterized in that** it is a cosmetic composition that contains, besides the 1,3,5-triazine derivative, at least one other additional UV-A-active and/or UV-B-active organic screening agent.

16. Composition according to Claim 15, **characterized in that** the organic UV screening agent (s) is (are) chosen from dibenzoylmethane derivatives, cinnamic derivatives, anthranilates; salicylic derivatives, camphor derivatives, benzophenone derivatives; β,β-diphenylacrylate derivatives, benzotriazole derivatives; benzalmalonate derivatives; benzimidazole derivatives; imidazolines; bis-benzazolyl derivatives; p-aminobenzoic acid (PABA) derivatives; methylene-bis(hydroxyphenyl)benzotriazole derivatives, screening polymers and screening silicones; dimers derived from α-alkylstyrene and 4,4-diarylbutadienes.

17. Composition according to Claim 16, **characterized in that** the organic UV screening agent (s) is (are) chosen from the following compounds:
- Ethylhexyl Salicylate,
- Octocrylene,
- Phenylbenzimidazole Sulfonic Acid,
- 4-Methylbenzylidene camphor,
- Benzimidazilate,
- Terephthalylidene Dicamphor Sulfonic,
- Benzophenone-3,
- Benzophenone-4,
- Benzophenone-5,
- Methylene bis-Benzotriazolyl Tetramethylbutylphenol,
- Drometrizole Trisiloxane
- and mixtures thereof.

18. Composition according to one of the preceding claims, **characterized in that** it also comprises nacres and coated or uncoated metal oxide pigments or nanopigments.

19. Composition according to Claim 18, **characterized in that** the said pigments or nanopigments are chosen from titanium oxide, zinc oxide, iron oxide, zirconium oxide and cerium oxide, and mixtures thereof, which may be coated or uncoated.

20. Composition according to one of the preceding claims, **characterized in that** it also comprises at least one adjuvant chosen from fatty substances, organic solvents, emulsifiers, ionic or nonionic thickeners, softeners, antioxidants, free-radical scavengers, opacifiers, stabilizers, emollients, silicones, α-hydroxy acids, antifoams, moisturizers, vitamins, insect repellents, fragrances, preserving agents, surfactants, anti-inflammatories, substance P antagonists, fillers, polymers, propellants, acidifying or basifying agents, and colorants.

21. Composition according to one of the preceding claims, **characterized in that** it is a composition for protecting the human epidermis or an antisun composition and **in that** it is in the form of a nonionic vesicular dispersion, an emulsion, in particular an emulsion of water-in-oil or oil-in-water type, a cream, a triple emulsion (W/O/W or O/W/O), a milk, a gel, a cream-gel, a suspension, a dispersion, a powder, a solid tube, a mousse or a spray.

22. Composition according to one of Claims 1 to 20, **characterized in that** it is a makeup composition for the eyelashes, the eyebrows or the skin and **in that** it is in solid or pasty, anhydrous or aqueous form or in the form of an emulsion, a suspension or a dispersion.

23. Composition according to one of Claims 1 to 20, **characterized in that** it is a composition for protecting the hair against ultraviolet rays and **in that** it is in the form of a shampoo, a lotion, a gel, an emulsion or a nonionic vesicular dispersion.

24. Use of a composition according to one of the preceding claims in or for the manufacture of cosmetic or dermatological compositions for protecting the skin and/or the lips and/or integuments against ultraviolet radiation, in particular solar radiation.

25. Cosmetic treatment process, **characterized in that** it consists in applying a composition according to one of Claims 1 to 21 to the skin and/or the lips and/or integuments.

26. Use of at least one N-acyl amino acid ester of formula
R'₁(CO)N(R'₂)CH(R'₃) (CH₂)ₙ(CO)OR'₄
in which:
n is an integer equal to 0, 1 or 2,
R'₁ represents a linear or branched C₅ to C₂₁ alkyl or alkenyl radical,
R'₂ represents a hydrogen atom or a C₁ to C₃ alkyl group,
R'₃ represents a radical chosen from the group formed by a hydrogen atom, a methyl group, an ethyl group and a linear or branched C₃ or C₄ alkyl chain,
R'₄ represents a linear or branched C₁ to C₁₀ alkyl radical or a linear or branched C₂ to C₁₀ alkenyl radical or a sterol residue, in antisun compositions containing a 1,3,5-triazine derivative, in order to improve the sun protection factor of this composition.
